# EUROPÄISCHE PATENTSCHRIFT

(11) **EP 1 293 555 B1**
(45) Veröffentlichungstag und Bekanntmachung des Hinweises auf die Patenterteilung: **07.06.2006**
(21) Anmeldenummer: 02020195.0
(22) Anmeldetag: 10.09.2002
(51) Int. Cl.: C11B 9/00, C07C 33/14

(54) **Duft- und Aromastoff**
Perfuming compounds
Composés parfumés

(30) Priorität: 12.09.2001 DE 10144816
(43) Veröffentlichungstag der Anmeldung: 19.03.2003
(73) Patentinhaber: Symrise GmbH & Co. KG, 37603 Holzminden (DE)
(72) Erfinder: Kappey, Klaus-Hermann, Dr., 37603 Holzminden (DE); Hölscher, Bernd, 37620 Halle (DE); Meier, Manfred, 37699 Fürstenberg (DE); Braun, Norbert Andreas, Dr., 37603 Holzminden (DE); Weber, Berthold, Dr., 37603 Holzminden (DE); Pickenhagen, Wilhelm, Dr., 37671 Höxter (DE)
(74) Vertreter: Eisenführ, Speiser & Partner

(56) Entgegenhaltungen:
- WO-A-86/03737
- DE-A- 3 245 524
- GB-A- 2 020 656
- US-A- 4 057 515
- US-A- 4 229 324
- US-A- 4 762 819

## Beschreibung

Die Erfindung betrifft eine Verbindung der Formel (2R/S, 3R/S)-3-Methyl-4-[(E,1R,2R,6R,7R)tricyclo[5.2.1.0^{2,6}]dec-4-en-8-yliden]butan-2-ol, wobei jeweils unabhängig voneinander gilt:
- (2R/S, 3R/S) bedeutet (2R,3R), (2R,3S), (2S,3R) oder (2S,3S).

Die Erfindung betrifft ferner bevorzugte Konfigurationsisomere (Diastereomere, Enantiomere) der genannten Verbindung mit besonders ausgeprägten sensorischen Eigenschaften sowie entsprechende Gemische.

WO8603737 und US4762819 offenbaren Tricyclodecenylalkohol Derivate und ihre Verwendung als Duft- oder Aromastoff.

Die Erfindung betrifft zudem Duft- und/oder Aromastoffkompositionen, die eine sensorisch (organoleptisch) aktive (wirksame) Menge der genannten Verbindung (einschließlich der zugehörigen Konfigurationsisomeren) oder eines entsprechenden Konfigurationsisomeren-Gemisches umfassen.

Die Erfindung betrifft auch Verfahren zum Modifizieren der sensorischen Eigenschaften einer Duftstoff- oder Aromakomposition, wobei eine oder mehrere Komponenten der Duftstoff- oder Aromakomposition mit einer sensorisch wirksamen Menge der erfindungsgemäßen Verbindung (einschließlich der zugehörigen Konfigurationsisomeren) oder eines entsprechenden Gemisches vermischt werden.

In der parfümistischen und flavoristischen Praxis besteht generell ein beständiger Bedarf an synthetischen Duft- und Aromastoffen, die sich günstig und mit gleichbleibender Qualität herstellen lassen, bei längerer Lagerung möglichst auch im Kontakt mit anderen Stoffen stabil bleiben und erwünschte olfaktorische bzw. geschmackliche Eigenschaften haben. Duftstoffe sollen angenehme, möglichst naturnahe Duftnoten von ausreichender Intensität aufweisen und in der Lage sein, den Duft von kosmetischen oder technischen Konsumgütern vorteilhaft zu beeinflussen. Aromastoffe sollen gut verträglich sein, an typische Geschmackskomponenten beliebter Speisen erinnern oder sogar mit diesen identisch sein und dazu beitragen können, den Geschmack von Lebensmitteln, oral zu verabreichenden Medikamenten und dgl. positiv zu beeinflussen. Das Auffinden von Duft- und Aromastoffen, die diesen Anforderungen entsprechen, hat sich als verhältnismäßig aufwendig erwiesen und erfordert regelmäßig umfangreiche Untersuchungen, insbesondere, wenn interessante neuartige Duftnoten oder Geschmacksrichtungen angestrebt werden.

Die Suche nach geeigneten Duft- oder Aromastoffen wird für den Fachmann insbesondere durch folgende Sachverhalte erschwert:
- Die Mechanismen der Duft- bzw. Aromawahrnehmungen sind nicht bekannt.
- Eine objektiv-quantitative Charakterisierung eines Duftes oder Aromas ist nicht möglich.
- Die Zusammenhänge zwischen der Duft- und/oder Aromawahrnehmung einerseits und der chemischen Struktur des Duft- und/oder Aromastoffs andererseits sind nicht hinreichend erforscht.
- Häufig bewirken bereits geringfügige Änderungen am strukturellen Aufbau bekannter Duft- oder Aromastoffe starke Änderungen der olfaktorischen bzw. geschmacklichen Eigenschaften und führen zu einer Beeinträchtigung der Verträglichkeit für den menschlichen Organismus.

Der Erfolg der Suche nach geeigneten Duft- oder Aromastoffen hängt deshalb regelmäßig von der Intuition des Suchenden ab.

Der vorliegenden Erfindung liegt die Aufgabe zugrunde, unter Beachtung der vorstehend beschriebenen generellen Rahmenbedingungen einen Duft- und Aromastoff anzugeben, welcher insbesondere in der Lage ist, üblichen Duftstoff- oder Aroma-Grundkompositionen einen an natürliches Sandelholz erinnernden Duft zu vermitteln oder den vorhandenen Duft derartiger Kompositionen in vorteilhafter Weise zu modifizieren.

Die anzugebenden Substanzen sollten dem Parfümeur oder Flavoristen beim Komponieren von Duftstoffen oder Aromen eine vielseitig einsetzbare Alternative zu den bislang eingesetzten oder beschriebenen Duftstoffen mit sandelartigem oder sandelig-holzigem Duft bieten. Beim schöpferischen Vorgang des Komponierens, einem langwierigen Prozess, der in der Regel nur von Spezialisten durchgeführt wird, genügt es zum Erreichen eines in der Vorstellung bereits existierenden Duft- oder Aromabildes nämlich nicht, schablonenartig einen beliebigen Duft- oder Aromastoff einzusetzen, dem in der Literatur ein bestimmter Geruchs- oder Aromaaspekt zugeordnet wurde. Die Geruchs- oder Aromacharakteristik einer Komposition lässt sich nämlich nicht im Sinne einer Addition präzise vorhersagen, wenn nur die Bestandteile der Komposition bekannt sind, denn diese Bestandteile unterliegen im Gemisch unvorhersehbaren Wechselwirkungen. Wichtig ist deshalb auch die Kompatibilität eines Duft- oder Aromastoffs mit den weiteren Bestandteilen einer Komposition und das Vorhandensein oder Fehlen von begleitenden sensorisch wahrnehmbaren Aspekten, die den Gesamtcharakter der fertigen Komposition (mit-)beeinflussen, ohne dass sie vielleicht in der Geruchsbeschreibung der reinen Substanz eine besondere Würdigung erfahren.

Die Erfindung beruht auf der überraschenden Erkenntnis, dass die erfindungsgemäße Verbindung (2R/S, 3R/S)-3-Methyl-4-(E,1R,2R 6R,7R)tricyclo-[5.2.1.0^{2,6}]dec-4-en-8-yliden]butan-2-ol gemäß nachstehender Formel rac-1 (Darstellung ohne Angabe der absoluten Stereochemie; rac = racemisch) und deren Konfigurationsisomere gemäß nachstehenden Formeln A, B, C, D, zur Verwendung als Duft- oder Aromastoff sowie zur Lösung der vorstehend angegebenen Aufgaben hervorragend geeignet ist.

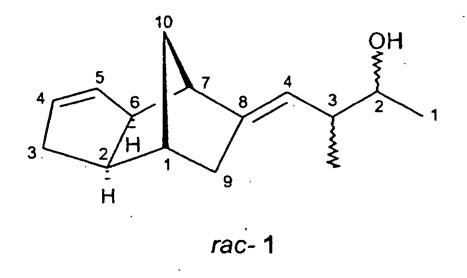

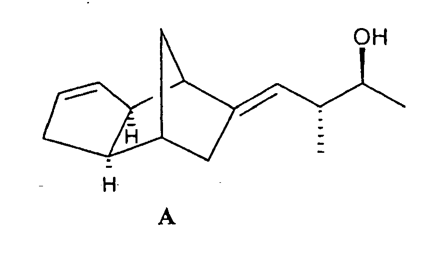

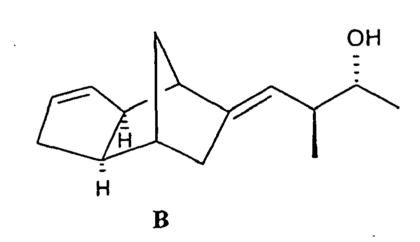

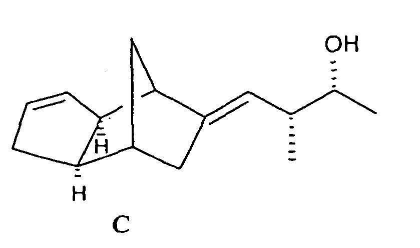

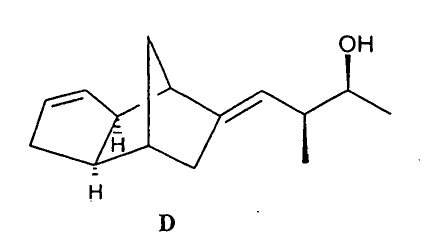

Die dargestellten Konfigurationsisomeren der erfindungsgemäßen Verbindung besitzen jeweils einen sehr interessanten Geruch, denn die Verbindungen mit dem Konfigurationsmerkmal (E,1 R,2R,6R,7R) besitzen einen deutlich sandelartigen Geruch, der im Einzelfall durch Nebenaspekte gefärbt ist, während nicht - erfindungsgemäße Konfigurationsisomere mit dem Konfigurationsmerkmal (E,1S,2S,6S,7S) einen Geruch besitzen, welcher als eher holzig zu beschreiben ist.

Unter den erfindungsgemäßen Konfigurationsisomeren mit dem Konfigurationsmerkmal (E,1R,2R,6R,7R) sind die Verbindungen mit dem Konfigurationsmerkmal (2S, 3R), d.h. die Verbindung gemäß Formel A, oder (2S,3S), d.h. die Verbindung gemäß Formel D, wegen ihrer überraschenden geruchlichen Nebennoten von besonderer Bedeutung für die Kreation neuer Duftstoffkompositionen. Dies wird weiter unten in den Beispielen noch näher erläutert.

Die erfindungsgemäße Verbindung (repräsentiert durch ihre 4 Konfigurationsisomeren) gehört zu einer größeren Gruppe von Verbindungen, welche in der WO86/03737 beschrieben ist. Die in Beispiel 2 dieser Schrift offenbarte Verbindung 9-(2-Methyl-3-hydroxy-butyliden)-2,6-exo-tricyclo[5.2.1.0^{2,6}]-decen-3(4) umfasst insgesamt 32 Isomere, die sich hinsichtlich der Position der Doppelbindung im endständigen Fünfring (Kohlenstoffatome C2 bis C6), hinsichtlich der Konfiguration an der Doppelbindung zwischen dem C8 der Ringstruktur und dem C4 der Seitenkette (E oder Z-Konfiguration) und hinsichtlich ihrer Stereochemie (Konfiguration an C2 und C3 in der Seitenkette sowie Konfiguration der chiralen Zentren in der Ringstruktur) unterscheiden können. In der WO86/03737 finden sich jedoch keine Hinweise auf die enfindungsgemäßen Konfigurationsisomeren, sondern es ist immer nur die Rede von der genannten Verbindung in ihrer allgemeinen Form. Unter Verwendung der im Rahmen dieses Textes ansonsten verwendeten Nomenklatur lässt sich die aus der WO86/03737 bekannte Verbindung durch die Bezeichnungen 3-Methyl-4-[(E/Z,RS,2RS,6RS,7RS)tricyclo[5.2.1.0^{2,6}]dec-4-en-8-yliden]butan-2-ol und 3-Methyl-4-[(E/Z,1RS,2SR,6RS,7RS)tricyclo[5.2.1.0^{2,6}]dec-3-en-8-yliden]butan-2-ol wiedergeben (wobei gilt: E/Z bedeutet E oder Z). Überraschenderweise hat sich gezeigt, dass unter den 32 Isomeren der aus der WO86/03737 bekannten Verbindung nur die erfindungsgemäßen Substanzen einen im Vordergrund stehenden Sondergeruch besitzen. Die insgesamt 24 Verbindungen der Formeln
3-Methyl-4-[(E,1R/S,2S/R,6R/S,7R/S)-tricyclo[5.2.1.0^{2,6}]dec-3-en-8-yliden]-butan-2-ol (Formel rac-2) 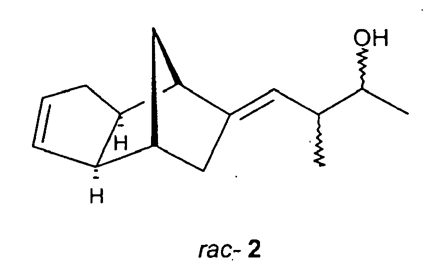 3-Methyl-4-[(Z,1R/S,2R/S,6R/S,7R/S)tricyclo[5.2.1.0^{2,6}]dec-4-en-8-yliden]-butan-2-ol (Formel rac-3) 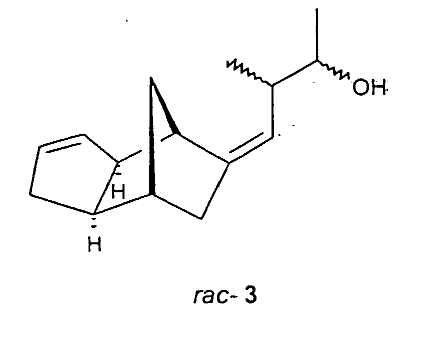 und
3-Methyl-4-[(Z,1R/S,2S/R,6R/S,7R/S)tricyclo[5.2.1.0^{2,6}]dec-3-en-8-yliden]-butan-2-ol (Formel rac-4) 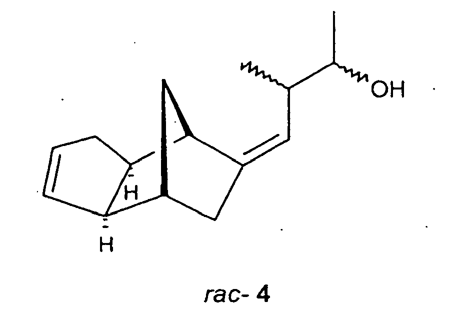 welche ebenfalls Vertreter der aus der WO86/03737 bekannten Verbindung sind, sind hingegen sensorisch uninteressant. Will der Fachmann daher z.B. zur Modifizierung einer Duftstoffkomposition nicht von einem oder mehreren reinen Konfigurationsisomeren der erfindungsgemäßen Verbindung ausgehen, sondern von einem in der Praxis kostengünstig erhältlichen Gemisch verschiedener Isomere, so wird er Sorge tragen, dass die sensorischen Eigenschaften des Gemisches überwiegend durch das oder die Konfigurationsisomeren der erfindungsgemäßen Verbindung bestimmt werden. Die ggf. anwesenden Verbindungen aus der Gruppe der 24 sensorisch uninteressanten Isomeren (siehe oben) sollen also sensorisch in den Hintergrund treten. Bei Verwendung der erfindungsgemäßen Konfigurationsisomere mit dem Konfigurationsmerkmal (E, 1 R, 2R, 6R. 7R), welche sich durch ihren sandelartigen Geruch auszeichnen, lassen sich vorgegebene Duftstoffkompositionen sehr effektiv sensorisch modifizieren. Es sei darauf hingewiesen, dass die in den Beispielen 8 und 10 der WO86/03737 offenbarten Modifizierungsverfahren unter Verwendung der (insgesamt 32 Konfigurationsisomeren umfassenden) Verbindung 3-Methyl-4-[E/Z-tricyclo[5.2.1.0^{2,6}]dec-3/4-en-8-yliden]butan-2-ol nicht zu einer Modifizierung führen, wie man sie durch Einsatz einer sensorisch-aktiven Menge einer erfindungsgemäßen Verbindung mit dem Konfigurationsmerkmal (E, 1 R, 2R, 6R, 7R) erhält.

Die Erfindung wird nachfolgend anhand von Beispielen näher erläutert.

### Beispiel 1: Synthesevorschriften (vgl. die beigefügten Schemata 1-11)

### 1.1 Synthesen der cyclischen Bausteine

### Alkoholgemisch rac-6/rac-7 (Schema 1)

Die Darstellung erfolgte aus endo-Dicyclopentadien (*rac*-**5**) nach H.A. Bruson, T.W. Riener (J. Am. Soc. Chem. 67, 723 (1945))

### Acetatgemisch rac-8/rac-9 (Schema 1)

75 g (0,5 mol) Alkoholgemisch *rac*-**6**/*rac*-**7**, 150 ml Toluol und 6 g Natriumcarbonat wurden unter Rühren und Rückfluß mit 60 g (0,59 mol) Essigsäureanhydrid versetzt. Nach 1 h wurde der Ansatz bei 80°C mit 100 g Wasser versetzt und weitere 15 min gerührt. Die wässerige Phase wurde abgetrennt; die organische Phase wurde mit Sodalösung neutralgewaschen und am Rotationsverdampfer vom Toluol befreit. Es verblieben 94 g Acetatgemisch, das gemäß ¹H-NMR-Spektrum die Isomere *rac*-**8** und *rac*-**9** im Verhältnis 3 : 2 enthielt.

Spektrale Daten der reinen Isomere siehe unten.

### Oxoacetate rac-10 und rac-11 (Schema 1)

In eine Lösung von 156,7 g (0,816 mol) Acetatgemisch *rac*-**8**/*rac*-**9** in 1 I Essigsäure und 500 ml Essigsäureanhydrid wurden unter Rühren 395 g (2,438 mol) Natriumchromat eingetragen. Die resultierende Lösung wurde 3 d bei 30 - 35°C gerührt. Das Reaktionsgut wurde in 500 ml Toluol und 3 I Wasser aufgenommen; die organische Phase wurde mit Sodalösung neutralgewaschen und bei 15 hPa vom Lösungsmittel befreit. Es verblieben 165 g Rohprodukt, die über eine 30 cm - Vigreux-Kolonne destilliert wurden; nach einem Vorlauf von 59,3 g Acetatgemisch *rac*-**8**/*rac*-**9** fielen im Siedebereich 110 -118°C bei 0,5 hPa 84,6 g Oxoacetatgemisch *rac*-**10**/*rac*-**11** als farblose Flüssigkeit an, die gemäß Gaschromatogramm (30 m DBWax, 100 - 240°C 6°C/min) die Isomere *rac*-**10** und *rac*-**11** im Verhältnis 55 : 45 enthielt. Durch erneute Destillation an einer 1 m - Drehbandkolonne wurde eine Fraktion (48,3 g) erhalten, in der das leichter siedende Isomer *rac*-**10** auf 93 % nach GC-Analyse angereichert war; während das Isomer *rac*-**11** zu 95 % angereichert im Rückstand (33,2 g) vorlag; beide Materialien erstarrten beim Abkühlen. Wiederholte Kristallisation aus tert.-Butyl-methylether lieferte reines *rac*-**10** vom Schmelzpunkt 119 - 119,5°C und reines *rac*-**11** vom Schmelzpunkt 84,5 - 85,5°C als farblose Kristalle.
Essigsäure-(1RS,2RS,6SR,7RS,8SR)-3-oxotricyclo[5.2.1.0^{2,6}]dec-4-en-8-ylester (*rac-*10):
¹H-NMR (300 MHz, CDCl₃): δ = 1.06 (dm, J = 11 Hz, 1 H), 1.36 (dm, J = 11 Hz, 1 H), 1.57 (ddd, J = 13.5, 4.25, 2.75 Hz, 1 H), 1.86 (ddd, J = 13.5, 7, 2.5 Hz, 1 H), 2.04 (s, 3 H), 2.12 (d, J = 5 Hz, 1 H), 2.34 (s, 1 H), 2.48 (d, J = 4.25 Hz, 1 H), 2.75 - 2.81 (m, 1 H), 4.75 (d,
J = 7 Hz, 1 H), 6.32 (dd, J = 5.5, 1.5 Hz, 1 H), 7.53 ppm (dd, J = 5.5, 2.75 Hz, 1 H). ¹³C-NMR (75 MHz, CDCl₃): δ = 21.2 (q), 28.3 (t), 37.7 (d), 38.2 (t), 43.2 (d), 46.1 (d), 52.2 (d), 76.7 (d), 137.7 (d), 164.1 (d), 170.6 (s), 210.3 ppm (s).
MS: m/z (%) = 206 (1, M⁺), 147 (11), 146 (100), 145 (14), 120 (17), 118 (19), 117 (18), 95 (47), 91 (17), 66 (11), 43 (38).
Essigsäure-(1RS,2SR,6RS,7RS,8SR)-5-oxotricyclo[5.2.1.0^{2,6}]dec-3-en-8-ylester (*rac*-**11**):
¹H-NMR (300 MHz, CDCl₃): δ = 1.09 (dm, J = 11 Hz, 1 H), 1.39 (dm, J = 11 Hz,1 H), 1.59 (ddd, J = 13.5, 4, 2.5 Hz, 1 H), 1.93 (ddd, J = 13.5, 7, 2.5 Hz, 1 H), 2.02 (s, 3 H), 2.14 (d, J = 5 Hz, 1 H), 2.30 (d, J = 4.5 Hz, 1 H), 2.47 (s, 1 H), 2.70 - 2.76 (m,1 H), 4.71 (d, J = 7 Hz, 1 H), 6.31 (dd, J = 5.5, 1.5 Hz, 1 H), 7.55 ppm (dd, J = 5.5, 2.5 Hz, 1 H).
¹³C-NMR (75 MHz, CDCl₃): δ = 21.2 (q), 28.2 (t), 36.9 (d), 39.3 (t), 43.7 (d), 48.4 (d), 49.9 (d), 75.6 (d), 137.6 (d), 165.2 (d), 170.4 (s), 209.9 ppm (s).
MS: m/z (%) = 206 (7, M⁺), 164 (29), 146 (100), 136 (29), 135 (21), 123 (30), 119 (24), 118 (21), 117 (27), 94 (28), 91 (30), 43 (45).

### Acetat rac-8 (Schema 1)

a) Eine Lösung von 14,75 g (0,071 mol) Oxoacetat *rac*-**11** in 200 g Ethanol (96 Vol.-%) wurde mit einer Spatelspitze Kupfer(I)-iodid versetzt und dann unter Rühren und Kühlen bei 20 - 25°C portionsweise in 30 min mit 1,86 g (0,049 mol) Natriumborhydrid versetzt. Der Ansatz wurde 45 min nachgerührt und dann durch vorsichtige Zugabe von 15 %iger Salzsäure hydrolysiert und neutralisiert. Nach Filtration wurde das Ethanol am Rotations-verdampfer abdestilliert; der Rückstand wurde in Diethylether und Wasser aufgenommen. Die wässerige Phase wurde abgetrennt; die organische Phase wurde mit Wasser gewaschen und am Rotationsverdampfer vom Lösungsmittel befreit. Es verblieben 14,25 g Essigsäure-(1 RS,2RS,6RS,7RS,8SR)-5-hydroxytricyclo[5.2.1.0^{2,6}]dec-8-ylester (*rac*-**12**); laut GC-Analyse lag kein Edukt mehr vor.
   MS (Hauptisomer): m/z (%) = 210 (Spur, M⁺), 150 (37), 132 (42), 121 (29), 109 (32), 106 (100), 105 (29), 91 (34), 79 (43), 67 (34), 66 (26), 43 (60).
b) Eine Lösung von 14 g (0,066 mol) des vorstehenden Hydroxyacetates und 1,2 g 4-Dimethyl-aminopyridin in 40 ml abs. Dichlormethan und 14 ml abs. Pyridin wurde bei 0 - 5°C unter Rühren mit 8,9 g (0,078 mol) Methansulfonsäurechlorid versetzt und über Nacht bei 20°C nachgerührt. Der Ansatz wurde mit 50 ml Wasser versetzt, 1 h gerührt und dann mit Dichlor-methan extrahiert. Die organische Phase wurde mit 10 %iger Schwefelsäure gewaschen, mit Sodalösung neutralgewaschen und am Rotationsverdampfer vom Lösungsmittel befreit. Es verblieben 19 g roher Methansulfonsäureester von Essigsäure-(1RS,2RS,6RS,7RS,8SR)-5-hydroxytricyclo[5.2.1.0^{2,6}]dec-8-ylester; laut DC-Analyse (Cyclohexan/Ethylacetat 4 : 1) lag kein Edukt mehr vor.
c) Eine Lösung von 14,4 g (0,05 mol) des vorstehenden Mesylates in 150 ml abs. Xylol (Isomerengemisch), 1 g getrocknetes Lithiumbromid und 15 g 1,8-Diazabicyclo[5.4.0]undec-7-en wurden 3 h unter Rückfluß gerührt. Das Reaktionsgut wurde mit Wasser und mit 10 %iger Schwefelsäure gewaschen und dann mit Sodalösung neutralgewaschen. Xylol wurde an einer 20 cm - Vigreux-Kolonne abdestllliert. Der Rückstand (9,4 g), der laut DC-Analyse (Cyclohexan/Ethylacetat 4 : 1) kein Edukt mehr enthielt, wurde an einer Mikro-Vigreux-Kolonne destilliert. Bei 114 - 115°C und 2 hPa fielen 7,2 g Acetat *rac*-**8** als farblose Flüssigkeit an.

### Essigsäure-(1RS,2RS,6RS,7RS,8SR)tricyclo[5.2.1.0^{2,6}]dec-4-en-8-ylester (rac-8):

¹H-NMR (300 MHz, CDCl₃): δ = 1.315 (br. s, 2 H), 1.39 (ddd, J = 13.5, 4, 2.5 Hz, 1 H), 1.76 (dd, J = 13.5, 7 Hz, 1 H), 1.89 (dm, J =17.5 Hz, 1 H), 1.97 - 2.09 (m, 2 H), 2.005 (s, 3 H), 2.115 (s, 1 H), 2.50 - 2.62 (m, 2 H), 4.66 (dd, J = 7, 2.5 Hz, 1 H), 5.40 - 5.46 (m, 1 H), 5.66 - 5.72 ppm (m, 1 H).
¹³C-NMR (75 MHz, CDCl₃): δ = 21.4 (q), 28.8 (t), 39.1 (t), 39.3 (t), 41.9 (d), 42.9 (d), 45.9 (d), 50.9 (d), 77.5 (d), 130.9 (d),132.7 (d), 170.7 ppm (s).
MS: m/z (%) = 192 (40, M⁺), 124 (81), 117 (62), 105 (30), 91 (45), 83 (52), 82 (47), 67 (48), 66 (100), 43 (65).

### Keton rac-13 (Schema 1)

Die Darstellung von Keton *rac*-**13** erfolgte analog zur nachstehenden Darstellung von Keton *rac*-**15**, d. h. durch Verseifung von Acetat *rac*-**8** oder p Nitrobenzoat *rac*-**16** zu Alkohol *rac*-**6** und dessen nachfolgende Oxidation.
(1RS,2RS,6RS,7RS)tricyclo[5.2.1.0^{2,6}]dec-4-en-8-on (*rac*-**13**):
¹H-NMR (300 MHz, CDCl₃): δ = 1.52 (dm, J = 10.5 Hz, 1 H), 1.655 (dm, J = 10.5 Hz, 1 H), 1.87 (dd, J = 17.5, 4 Hz, 1 H), 1.99 - 2.10 (m, 2 H), 2.33 - 2.42 (m, 3 H), 2.71 (ddm, J = 17.5, 9.5 Hz, 1 H), 2.90 - 2.97 (m, 1 H), 5.42 - 5.48 (m, 1 H), 5.76 - 5.81 ppm (m, 1 H).
¹³C-NMR (75 MHz, CDCl₃): δ = 31.0 (t), 39.6 (t), 41.7 (d), 42.3 (d), 45.3 (t), 50.1 (d), 54.0 (d), 129.4 (d), 133.4 (d), 217.5 ppm (s).
MS: m/z (%) = 148 (100, M⁺), 105 (35), 104 (26), 92 (38), 91 (57), 79 (49), 78 (28), 77 (32), 67 (21), 66 (35).

### Acetat rac-9 (Schema 1)

Die Darstellung von Acetat *rac*-**9** erfolgte analog zur vorstehenden Darstellung von Acetat *rac*-**8**, d. h. aus Oxoacetat *rac*-**10** via Essigsäure
(1RS,2RS,6RS,7RS,8SR)-3-hydroxy-tricyclo[5.2.1.0^{2,6}]dec-8-ylester *(rac-14).*
Essigsäure-(1RS,2SR,6RS,7RS,8SR)tricyclo[5.2.1.0^{2,6}]dec-3-en-8-ylester (*rac-***9**):
¹H-NMR (300 MHz, CDCl₃): δ = 1.33 (br. s, 2 H), 1.44 (ddd, J = 13.5, 4.25, 2.5 Hz, 1 H), 1.81 (ddd, J = 13.5, 7, 2 Hz, 1 H), 1.92 (dm, J = 17 Hz, 1H), 2.005 (s, 3 H), 2.02 - 2.16 (m, 3 H), 2.535 (br. d, J = 7.5 Hz, 1 H), 2.62 (ddm, J = 17, 10 Hz, 1 H), 4.61 (dd, J = 7, 2.5 Hz, 1 H), 5.42 - 5.47 (m, 1 H), 5.65 - 5.71 ppm (m, 1 H). ¹³C-NMR (75 MHz, CDCl₃): δ = 21.4 (q), 28.9 (t), 38.9 (d), 39.15 (t), 39.2 (t), 39.5 (d), 48.3 (d), 54.9 (d), 76.9 (d), 131.9 (d), 132.3 (d), 170.8 ppm (s).
MS: m/z (%) = 192 (1, M'), 132 (48), 117 (22), 104 (12), 91 (21), 79 (14), 77 (15), 67 (31), 66 (100), 43 (42), 39 (14).

### Keton rac-15 (Schema 1)

a) Eine Lösung von 9,6 g (0,05 mol) Acetat *rac*-**9** in 50 g Methanol wurde mit 5 g 50 %iger Natronlauge 2 h unter Rückfluß gerührt. Das Methanol wurde am Rotationsverdampfer abdestilliert; der Rückstand wurde in 50 ml Wasser aufgenommen und mit Diethylether extrahiert. Die organische Phase wurde mit Wasser gewaschen und am Rotations-verdampfer vom Lösungsmittel befreit.
   Es verblieben 7,3 g Alkohol *rac*-**7**.
   MS: m/z (%) = 150 (32, M⁺), 132 (13), 117 (18), 106 (27), 91 (39), 83 (20), 79 (26), 78 (21), 77 (24), 67 (46), 66 (100), 39 (24).
b) Eine Lösung von 1,5 g (0,01 mol) des vorstehenden Alkohols in 100 ml Dichlormethan wurde mit 4,5 g Pyridiniumdichromat über Nacht bei 20 - 25°C gerührt. Das Reaktionsgut wurde über Kieselgel filtriert; das Filtrat wurde am Rotationsverdampfer vom Lösungsmittel befreit und bei 1 hPa am Kugelrohr destilliert. Laut GC-Analyse lag kein Edukt mehr vor. Es verblieben 1,3 g Keton *rac*-**15**.
(1RS,2SR,6RS,7RS)tricyclo[5.2.1.0^{2,6}]dec-3-en-8-on (*rac*-**15**):
H-NMR (300 MHz, CDCl₃): δ = 1.53 (dm, J = 10.5 Hz, 1 H), 1.745 (dm, J = 10.5 Hz, 1 H), 1.92 (dd, J = 17.5, 4 Hz, 1 H), 1.98 - 2.14 (m, 2 H), 2.33 (br. s, 1 H), 2.40 - 2.49 (m, 2 H), 2.70 (ddm, J = 17.5, 10 Hz, 1 H), 2.85 - 2.92 (m, 1 H), 5.55 - 5.60 (m, 1 H), 5.76 - 5.81 ppm (m, 1 H).
¹³C-NMR (75 MHz, CDCl₃): δ = 31.1 (t), 37.9 (d), 38.8 (t), 39.7 (d), 44.9 (t), 54.4 (d), 56.2 (d), 131.6 (d), 133.1 (d), 217.3 ppm (s).
MS: m/z (%) = 148 (100, M⁺), 105 (28), 104 (34), 92 (30), 91 (55), 82 (52), 79 (48), 78 (29), 77 (34), 66 (63).

### p-Nitrobenzoate rac-16 und rac-17 (Schema 2)

75 g (0,5 mol) Alkoholgemisch *rac*-**6**/*rac*-**7** wurden in 250 ml abs. Pyridin gelöst, unter Rühren bei 0 - 5°C in 30 min portionsweise mit 115 g (0,62 mol) 4-Nitrobenzoesäurechlorid versetzt und über Nacht bei 20°C nachgerührt. Der Ansatz wurde mit 300 g Wasser versetzt, 1h gerührt und dann in 300 ml Toluol aufgenommen. Die wässerige Phase wurde abgetrennt; die organische Phase wurde zweimal mit 150 g 10 %iger Schwefelsäure gewaschen, mit Sodalösung neutralgewaschen und am Rotationsverdampfer vom Toluol befreit. Es verblieben 149 g Rohprodukt als amorpher Feststoff, der gemäß ¹H-NMR-Spektrum die Isomere *rac*-**16** und *rac-***17** im Verhältnis 59 : 41 enthielt. Durch wiederholte fraktionierende Kristallisation aus tert.-Butyl-methylether wurde das im Überschuß vorliegende und bevorzugt kristallisierende Isomer *rac*-**16** vom Isomer *rac*-**17**, das sich in den Mutterlaugen anreicherte, abgetrennt. Es wurde reines *rac*-**16** vom Schmelzpunkt 134 - 134,5°C als farbloses Pulver erhalten.
4-Nitrobenzoesäure-(1RS,2RS,6RS,7RS,8SR)tricyclo[5.2.1.0^{2,6}]dec-4-en-8-ylester (*rac*-16):
¹H-NMR (300 MHz, CDCl₃): δ =1.43 ("tm", J = 11.5 Hz, 2 H), 1.59 (ddd, J = 13.5, 4, 2.5 Hz, 1 H), 1.86 - 1. 99 (m, 2 H), 2.08 -2.20 (m, 2 H), 2.30 (br. s, 1 H), 2.54 - 2.71 (m, 2 H), 4.96 (dd, J = 7, 2.5 Hz, 1 H), 5.44 - 5.50 (m, 1 H), 5.70 - 5.76 (m, 1 H), 8.18 (dm, J = 9 Hz, 2 H), 8.27 ppm (dm, J = 9 Hz, 2 H).
¹³C-NMR (75 MHz, CDCl₃): δ = 29.1 (t), 39.2 (t), 39.3 (t), 41.9 (d), 42.9 (d), 46.0 (d), 50.8 (d), 79.2 (d), 123.4 (2d), 130.6 (2d), 130.7 (d), 132.9 (d), 136.1 (s), 150.4 (s), 164.3 ppm (s).

### Alkohol 6 und Acetat ent-8 durch enzymatische Racematspaltung von'Acetat rac-8 (Schema 3)

5 g Lipase (*Porcine Pancreas* Type II; Fa. Sigma) wurden unter Rühren in 430 ml Phosphat-puffer (pH = 7) vorgelegt. Nach 30 min wurde die Suspension mit 1 N Natronlauge bzw. verdünnter Salzsäure auf pH = 7 eingestellt. Anschließend wurden 11,50 g (60 mmol) Acetat *rac*-**8** zugetropft und die Suspension 11 d bei 20 - 25°C nachgerührt. Während dieses Zeit-raums wurde die Suspension in regelmäßigen Abständen mit 1 N Natronlauge auf pH = 7 justiert und zusätzlich insgesamt 11 g Lipase nachgegeben. Die Umsetzung wurde gaschromatografisch verfolgt und bei einem Verhältnis von Alkohol zu Acetat von 55 : 45 abgebrochen. Nach Zugabe von 500 ml Wasser wurde dreimal mit je 300 ml Methyl-*tert*.-butylether extrahiert. Die vereinigten organischen Phasen wurden mit 5 %iger Natrium-chloridlösung gewaschen und dann über Natriumsulfat getrocknet. Nach Entfernen des Lösungsmittels im Vakuum wurde das erhaltene Rohprodukt an Kieselgel 60 (Fa. Merck; Cyclohexan/Ethylacetat 95 : 5 bis 80 : 20) chromatografiert. Es wurden 5,2 g (60 % ee) enantiomerenangereichertes Acetat *ent-***8** und 4,9 g (48 % ee) enantiomerenangereicherter Alkohol **6** erhalten.

Der Enantiomerenüberschuß (ee) an **6** wurde per chiraler Gaschromatografie (Vorsäule: DBWax, 30 m, 80 - 240°C 4°C/min; Hauptsäule: Ethyl-β-Bicchi, 15 min 80°C, - 200°C 1°C/min) ermittelt: t_{R} = 30.1 (74 % **6**), 30.3 min (26 % *ent*-**6**).
(1R,2R,6R,7R,8S)tricyclo[5.2.1.0^{2,6}]dec-4-en-8-ol (6):
¹H-NMR (300 MHz, CDCl₃): δ = 1.19 - 1.32 (m,2H), 1.38(ddd, J = 10.1, 1.4, 1.4 Hz, 1 H), 1.67 (ddd, 13.0, 7.0, 2.4 Hz, 1 H), 1.87 (dm, J = 17.2 Hz, 1 H), 1.96 (br. d, J = 1.3 Hz, 2H), 1.98 - 2.05 (m, 1 H), 2.40 - 2.47 (m, 1 H), 2.53 (ddddd, J = 17.0, 9.6, 2.2, 2.2, 1.3 Hz, 1H), 3.05 (br. s, OH), 3.79 (d, J = 6.6 Hz, 1H), 5.42 (dddd, J = 4.6, 2.2, 2.2, 2.2 Hz, 1H), 5.66 ppm (dddd, J = 5.9, 2.0, 2.0, 2.0 Hz, 1H).
¹³C-NMR (75 MHz, CDCl₃): δ = 28.0 (t), 39.2 (t), 41.7 (t), 42.0 (d), 42.9 (d), 48.7 (d), 51.2 (d), 74.5 (d), 131.2 (d), 132.2 ppm (d).
MS: m/z (%) = 150 (45, M⁺), 132 (38), 117 (78), 105 (31), 91 (60), 83 (42), 79 (30), 77 (32), 67 (34), 66 (100).

### Alkohol ent-6 (Schema 3)

5,2 g (27 mmol) des enantiomerenangereicherten Acetates *ent*-**8** wurden mit 5 ml 50 %iger Natronlauge und 35 ml Methanol 2 h unter Rühren und Rückfluß erhitzt. Nach Zugabe von 100 ml Wasser wurde dreimal mit je 50 ml Diethylether extrahiert. Die vereinigten organischen Phasen wurden mit 5 %iger Natriumchloridlösung gewaschen und über Natriumsulfat getrocknet. Die Lösungsmittel wurden am Rotationsverdampfer unter vermindertem Druck entfernt und das erhaltene Rohprodukt an Kieselgel 60 (Fa. Merck; Cyclohexan/ Ethylacetat
9 : 1 bis 8 : 2) chromatografiert. Man erhielt 4,06 g (60 % ee) enantiomerenangereicherten Alkohol *ent-***6***.*

Der Enantiomerenüberschuß an *ent*-**6** wurde per chiraler GC (siehe oben) ermittelt: t_{R} = 30.1 (20 % 6), 30.3 min (80 % *ent*-**6**).
(1S,2S,6S,7S,8R)Tricyclo[5.2.1.0^{2,6}]dec-4-en-8-ol (*ent*-**6**):
Die spektralen Daten entsprechen denen von Alkohol **6**.

### Camphansäureester ent-19 (Schema 3)

Zu einer Lösung von 4 g (27 mmol) des enantiomerenangereicherten Alkoholes *ent-***6** (60% ee) in 40 ml Pyridin wurden 5,96 g (28 mmol) (S)-(-)-Camphansäurechlorid (*ent*-**18**) sowie 40 mg 4-Dimethylaminopyridin hinzugefügt und 24 h bei 20 - 25°C nachgerührt. Nach Zugabe von 100 ml Wasser und Ansäuern mit 20 %iger Schwefelsäure wurde dreimal mit je 50 ml Diethyl-ether extrahiert. Die vereinigten organischen Phasen wurden mit 5 %iger Sodalösung sowie 5 %iger Natriumchloridlösung gewaschen und anschließend über Natriumsulfat getrocknet. Nach Entfernen des Lösungsmittels am Rotationsverdampfer wurde das erhaltene Diastereomerengemisch aus Diethylether umkristallisiert, wobei sich das bevorzugt auskristallisierende Hauptdiastereomer *ent*-**19** anreicherte (Die Diastereomerenreinheit wurde mit Hilfe der für das Nebendiastereomere signifikanten ¹³C-NMR-Signale bei 78.85 bzw. 167.0 ppm geprüft). Durch wiederholte Kristallisation erhielt man 4,31 g reinen Camphansäureester *ent*-**19** als farblose Nadeln vom Schmelzpunkt 127,5 - 128°C.

Die absolute Konfiguration von *ent*-**19** wurde per Röntgenstrukturanalyse eines Einkristalls ermittelt.
(1S,4R)-3-Oxo-4,7,7-trimethyl-2-oxabicyclo[2.2.1]heptan-1-carbonsäure-(1S,2S,6S,7S,8R)tricyclo[5.2.1.0^{2,6}]dec-4-en-8-ylester *(ent-19):*
¹H-NMR (300 MHz, CDCl₃): δ = 0.96 (s, 3H), 1.05 (s, 3H), 1.11 (s, 3H), 1.34 (s, 2H), 1.48 (ddd, J = 13.7, 4.2, 2.2 Hz, 1H), 1.67 (ddd, J = 13.7, 4.2, 2.2 Hz, 1H), 1.77 - 2.00 (m, 7H), 2.41 (ddd, J = 13.3, 10.5, 4.2 Hz, 1H), 2.50 - 2.70 (m, 2H), 4.83 (dd, J = 7.0, 2.2 Hz, 1H), 5.44 (dddd, J = 5.1, 2.4, 2.4, 2.4 Hz,1H), 5.70 ppm (dddd, J= 5.9, 2.0, 2.0, 2.0 Hz, 1H).
¹³C-NMR (75 MHz, CDCl₃): δ = 9.7 (q), 16.7 (q), 16.8 (q), 28.85 (t), 28.9 (t), 30.5 (t), 39.0 (t), 39.2 (t), 41.8 (d), 42.9 (d), 46.1 (d), 50.7 (d), 54.1 (s), 54.8 (s), 78.9 (d), 91.0 (s), 130.6 (d), 133.0 (d), 167.1 (s), 178.3 ppm (s).

### Camphansäureester 19 (Schema 3)

Analog zur Darstellung von *ent*-**19** wurden 2,32 g (15 mmol) enantiomerenangereicherter Alkohol 6 (48% ee) mit 3,4 g (16 mmol) (R)-(+)-Camphansäurechlorid (**18**) verestert. Nach Aufarbeitung wurde das erhaltene Diastereomerengemisch aus Diethylether umkristallisiert. Durch wiederholte Kristallisation wurden 2,77 g reiner Camphansäureester **19** als farblose Nadeln vom Schmelzpunkt 127,5 - 128°C erhalten
(1R,4S)-3-Oxo-4,7,7-trimethyl-2-oxabicyclo[2.2.1]heptan-1-carbonsäure-(1R,2R,6R.7R,8S)tricyclo[5.2.1.0^{2,6}]dec-4-en-8-ylester (**19**):

Die spektralen Daten entsprechen denen von Ester *ent*-**19**.

### Keton ent-13 (Schema 3)

a) Eine Lösung von 4,31 g (13 mmol) diastereomerenreinem Camphansäureester *ent*-**19** in 50 ml Methanol wurde mit 5 g 50 %iger Natronlauge versetzt und 2 h unter Rühren und Rückfluß erhitzt. Nach Zugabe von 100 ml Wasser wurde dreimal mit je 50 ml Diethylether extrahiert. Die vereinigten organischen Phasen wurden mit 5 %iger Natriumchloridlösung gewaschen und anschließend über Natriumsulfat getrocknet. Nach Entfernen der Lösungsmittel verblieben 1,95 g (> 98 % ee) roher Alkohol *ent*-**6**. Der Enantiomerenüberschuß an *ent*-**6** wurde per chiraler GC (siehe oben) ermittelt: t_{R} = 30,1 (< 1 % 6), 30,3 min (> 99 % *ent*-**6**).
b) Eine Lösung von 1,95 g des vorstehenden rohen Alkohols in 100 ml Dichlormethan wurde unter Rühren portionsweise mit insgesamt 6,77 g (18 mmol) Pyridiniumdichromat versetzt und dann 16 h bei 20 - 25°C nachgerührt. Die Reaktionsmischung wurde über Kieselgel filtriert; das Filtrat wurde am Rotationsverdampfer vom Lösungsmittel befreit, und das Rohprodukt an Kieselgel 60 (Fa. Merck;Cyclohexan/Ethylacetat 9 : 1 bis 8 : 2) chromatografiert. Es wurden 1,74 g Keton *ent*-**13** erhalten.
(1S,2S,6S,7S,8R)Tricyclo[5.2.1.0^{2,6}]dec-4-en-8-on (*ent*-**13**):
Die spektralen Daten entsprechen denen von Keton *rac*-**13**.

### Keton 13 (Schema 3)

Analog zur Darstellung von *ent*-**13** wurden zunächst 2,77 g (8,4 mmol) diastereomerenreiner Camphansäureester **19** verseift. Nach Aufarbeitung verblieben 1,25 g (> 98 % ee) roher Alkohol **6.** Der Enantiomerenüberschuß an **6** wurde per chiraler GC (siehe oben) ermittelt: t_{R} = 30.1 (> 99 % **6**), 30.3 min (< 1 % *ent*-**6**).

Dann wurden 1,2 g des vorstehenden Rohalkoholes mit 3,76 g Pyridiniumdichromat (10 mmol) oxidiert. Nach Aufarbeitung und Chromatografie an Kieselgel wurden 1,03 g Keton 13 erhalten.
(1R,2R,6R,7R,8S)tricyclo[5.2.1.0^{2,6}]dec-4-en-8-on (**13**):
Die spektralen Daten entsprechen denen von Keton *rac*-**13**.

### 1.2 Synthese der Seitenketten-Bausteine

### 3-Hydroxy-2-methylen-buttersäurebutylester (rac-20) (Schema 4)

Die Darstellung erfolgte nach S.E. Drewes, N.D. Emslie (J. Chem. Soc., Perkin Trans. 1, 2079 (1982)).
3-Hydroxy-2-methylen-buttersäurebutylester (rac-**20**):
¹H-NMR (300 MHz, CDCl₃): δ = 0.955 (t, J = 7.5 Hz, 3 H), 1.355 (d, J = 6.5 Hz, 3 H), 1.36 - 1.48 (m, 2 H), 1.62 - 1.72 (m, 2 H), 3.53 (br. s, 1 H), 4.175 (t, J = 6.5 Hz, 2 H), 4.62 (q, J = 6.5 Hz, 1 H), 5.855 (t, J = 1.25 Hz, 1 H), 6.20 ppm (dd, J = 1.25, 0.75 Hz, 1 H).
¹³C-NMR (75 MHz, CDCl₃): δ = 13.7 (q), 19.3 (t), 22.5 (q), 30.7 (t), 64.7 (t), 66.6 (d), 123.6 (t), 144.4 (s), 166.7 ppm (s).
MS: m/z (%) = 157 (35, M⁺ - 15), 101 (100), 99 (51), 98 (46), 83 (53), 73 (44), 55 (54), 43 (56), 41 (69), 29 (96), 27 (79).

### (2RS,3RS)-3-Hydroxy-2-methyl-buttersäurebutylester (rac-21) (Schema 4)

25 g (0,145 mol) 3-Hydroxy-2-methylen-buttersäurebutylester (*rac*-**20**) wurden in 100 ml abs. Dichlormethan gelöst und mit Stickstoff begast. Nach Zugabe von 0,1 g Rhodium(I)-[1,4-bis-(diphenylphospino)-butan]-(2,5-norbornadien)-tetrafluoroborat (Fa. Aldrich) wurde die Lösung 3 h bei 0,5 - 1 MPa und 20 - 25°C in einer Wasserstoffatmosphäre gerührt. Das Reaktionsgut wurde über Talkum-Pulver filtriert, am Rotationsverdampfer vom Lösungsmittel befreit und bei 1 hPa am Kugelrohr destilliert. Es verblieben 24,7 g Produkt, das gemäß GC/MS-Analyse
den anti-Ester *rac*-**21** mit 98 % de enthielt.
(2RS,3RS)-3-Hydroxy-2-methyl-buttersäurebutylester (rac-**21**):
¹H-NMR (300 MHz, CDCl₃): δ = 0.94 (t, J = 7.5 Hz, 3 H), 1.165 (d, J = 7 Hz, 3 H), 1.205 (d, J = 6.5 Hz, 3 H), 1.32 - 1.46 (m, 2 H), 1.58 - 1.68 (m, 2 H), 2.465 (dq, J = 7, 7 Hz, 1 H), 3.26 (br. s, 1 H), 3.905 (dq (5 Linien), J = 6.5, 7 Hz, 1 H), 4.11 ppm (t, J = 6.5 Hz, 2 H).
¹³C-NMR (75 MHz, CDCl₃): δ = 13.7 (q), 13.8 (q), 19.2 (t), 20.5 (q), 30.7 (t), 47.2 (d), 64.4 (t), 69.3 (d), 175.9 ppm (s).
MS: m/z (%) = 159 (4, M⁺ - 15), 130 (13), 103 (13), 101 (61), 74 (100), 73 (13), 57 (30), 56 (34), 45 (16), 41 (13).

### Tetrahydropyranylether (rac-23) von (2RS,3RS)-4-Brom-3-methylbutan-2-ol (Schema 4)

a) Eine Lösung von 17,4 g (0,1 mol) (2RS,3RS)-3-Hydroxy-2-methylbuttersäurebutylester (*rac-***21**) in 100 ml abs. Diethylether wurde unter Rühren bei 20 - 25°C mit p-Toluolsulfon-säure und dann mit 10 g (0,12 mol) 3,4-Dihydro-2H-pyran versetzt. Nach 4 h wurde das Reaktionsgut mit Sodalösung gewaschen und am Rotationsverdampfer vom Lösungs-mittel befreit. Laut GC-Analyse lag kein Edukt mehr vor. Es verblieben 25,1 g roher Tetrahydropyranylether von (2RS,3RS)-3-Hydroxy-2-methyl-buttersäurebutylester.
   MS (beispielhaftes Diastereomer): m/z (%) = 257 (Spur, M⁺ - 1), 157 (11), 101 (63), 83 (10), 57 (16), 56 (10), 55 (9), 41 (10).
b) Eine Lösung von 25 g des vorstehenden rohen Esters in 50 ml abs. Tetrahydrofuran wurde unter Rühren bei 0 - 10°C zu einer Suspension von 2,3 g (0,06 mol) Lithiumalanat in 200 ml abs. Tetrahydrofuran getropft. Dann wurde 1 h unter Rückfluß nachgerührt. Der Überschuß an Lithiumalanat wurde bei 0°C vorsichtig mit Wasser zersetzt; dann wurde der Ansatz über Theorit filtriert und am Rotationsverdampfer vom Lösungsmittel befreit. Laut GC-Analyse lag kein Edukt mehr vor. Es resultierten 17,9 g rohes (2SR,3RS)-2-Methyl-3-(tetrahdro-2H-pyran-2-yloxy)butan-1-ol (*rac*-**22**; Schema 4).
   MS (beispielhaftes Diastereomer): m/z (%) = 187 (Spur, M⁺ - 1), 129 (7), 101 (19), 85 (100), 69 (12), 67 (10), 57 (13), 56 (25), 45 (12), 43 (10), 41 (16).
c) Eine Lösung von 9,4 g des vorstehenden rohen Alkohols (*rac*-**22**) in 100 ml abs. Toluol, 12,5 g Triethylamin und 1 g N,N,N',N'-Tetramethyl-hexan-1,6-diamin wurden unter Rühren bei 0°C mit 12,5 g (0,06 mol) p-Toluolsulfonsäurechlorid versetzt und über Nacht bei 20°C nachgerührt. Der Ansatz wurde mit 100 g Wasser versetzt und 1 h gerührt. Die wässerige Phase wurde abgetrennt; die organische Phase wurde mit 10 %iger Schwefelsäure gewaschen, mit Sodalösung neutralgewaschen und am Rotationsverdampfer vom Toluol befreit. Laut DC-Analyse (Cyclohexan/Ethylacetat 4 : 1) lag kein Edukt mehr vor. Es verblieben 17 g roher p-Toluolsulfonsäureester von (2SR,3RS)-2-Methyl-3-(tetrahdro-2H-pyran-2-yloxy)butan-1-ol.
d) Eine Lösung von 17 g des vorstehenden rohen Tosylates in 150 g abs. Aceton, 17,4 g frisch getrockenetes Lithiumbromid, 2,5 g Natriumcarbonat und 5 g Molekularsieb 4 Å wurden unter Rühren und Feuchtigkeitsausschluß 3 h refluxiert. Nach Abkühlung wurde der Ansatz über Theorit filtriert; das Filtrat wurde mit 150 ml Dichlormethan versetzt, erneut über Theorit filtriert und am Rotationsverdampfer vom Lösungsmittel befreit. Laut DC-Analyse (Cyclohean/Ethylacetat 4 : 1) lag kein Edukt mehr vor. Kugelrohr-Destillation des Rohproduktes (12,2 g) bei 1 hPa lieferte 10,5 g Bromid *rac*-**23**.

Nach NMR-Analyse lagen die beiden diastereomeren Tetrahydropyranylether
(*rac*-**23**) von (2RS,3RS)-4-Brom-3-methylbutan-2-ol im Verhältnis 1 : 1 vor:
¹H-NMR (300 MHz, CDCl₃): δ = 0.995 (d, J = 7 Hz, 1.5 H), 1.05 (d, J = 7 Hz, 1.5 H), 1.11 (d, J = 6 Hz, 1.5 H), 1.24 (d, J = 6 Hz, 1.5 H), 4.67 (dd, J = 4.5, 3 Hz, 0.5 H), 4.71 ppm (dd, J = 4, 3.5 Hz, 0.5 H).
¹³C-NMR (75 MHz, CDCl₃): δ = 14.9 (q), 15.3 (q), 15.9 (q), 18.6 (q), 18.8 (t), 19.7 (t), 25.5 (2t), 31.1 (2t), 38.2 (t), 38.6 (t), 40.8 (d), 41.4 (d), 62.6 (t), 63.0 (t), 72.5 (d), 77.0 (d), 95.2 (d), 100.5 ppm (d).
MS (beispielhaftes Diastereomer): m/z (%) = 251/249 (1/1, M⁺ - 1), 151/149 (5/5), 129 (13), 101 (19), 85 (100), 69 (38), 67 (10), 56 (18), 55 (11), 43 (11), 41 (23).

### (S)-(-)-3-Hydroxy-2-methylen-buttersäurebutylester (20) und

### (R)-(+)-3-Acetoxy-2-methylen-buttersäurebutylester (ent-24) (Schema 5)

Die Darstellung erfolgte nach K. Burgess, L.D. Jennings (J. Org. Chem. 55, 1138 (1990)). Per chiraler Gaschromatografie (Vorsäule: DBWax, 30 m, 80 - 240°C 4°C/min; Hauptsäule: Ethyl-β-Bicchi, 15 min 80°C, - 200°C 1°C/min) wurde die optische Reinheit der 3S-Verbindung **20** mit > 99,5 % ee und die der 3R-Verbindung *ent-***24** mit > 97,5 % ee ermittelt.

### (2S,3S)-3-Hydroxy-2-methyl-buttersäurebutylester (21) (Schema 5)

Darstellung durch Hydrierung von (S)-(-)-3-Hydroxy-2-methylenbuttersäurebutylester (**20**), analog wie vorstehend beschrieben.

### Tetrahydropyranylether (23) von (2S,3S)-4-Brom-3-methylbufan-2-ol (Schema 5)

Die Darstellung erfolgte analog zur vorstehend beschriebenen, vierstufigen Reaktionssequenz aus (2S,3S)-3-Hydroxy-2-methyl-buttersäurebutylester (**21**).

### (2R,3R)-3-Hydroxy-2-methyl-buttersäuremethylester (ent-27) (Schema 5)

a) 21,4 g (0,1 mol) (R)-3-Acetoxy-2-methylen-buttersäurebutylester (*ent* -**24**) wurden mit einer Lösung von 8,4 g Natriumhydroxid in 90 g Ethanol und 10 g Wasser unter Rühren 2 h refluxiert. Der Ansatz wurde am Rotationsverdampfer vom Ethanol befreit, in 150 ml Wasser aufgenommen und mit Diethylether gewaschen. Die wässerige Phase wurde mit 10 %iger Schwefelsäure auf pH = 2,5 eingestellt, mit Kochsalz gesättigt und dann mit Ethylacetat extrahiert. Nach Einengen dieses Extraktes am Rotationsverdampfer verblieben 11,1 g rohe (R)-3-Hydroxy-2-methylen-buttersäure (*ent*-**25**).
   ¹H-NMR (300 MHz, CDCl₃): δ = 1.40 (d, J = 6.5 Hz, 3 H), 4.69 (q, J = 6.5 Hz, 1 H), 5.96 (s, 1 H), 6.35 (s, 1 H), 8.22 ppm (br. s, 2 H).
   ¹³C-NMR (75 MHz, CDCl₃): δ = 22.1 (q), 66.7 (d), 126,2 (t), 143.1 (s), 170.5 ppm (s).
b) Eine Lösung von 11 g der vorstehenden rohen Säure in 200 ml abs. Diethylether wurde bei 0°C unter Rühren mit 350 ml einer frisch hergestellten etherischen Diazomethan-Lösung (ca. 0,12 mol CH₂N₂; Darstellung nach T.J. de Boer, H.J. Backer in Org. Synth. 36, 16 (1956)) versetzt und 15 min bei 20°C nachgerührt. Der Ansatz wurde mit 5 g Essigsäure versetzt, 5 min gerührt und dann in 100 ml Wasser aufgenommen. Die wässerige Phase wurde abgetrennt; die organische Phase wurde mit Sodalösung neutralgewaschen und am Rotationsverdampfer vom Lösungsmittel befreit. Kugelrohr-Destillation des rohen Methylesters (11,5 g) bei 1 hPa lieferte 10,9 g (R)-(+)-3-Hydroxy-2-methylen-buttersäuremethylester (*ent*-**26**).
   MS: m/z (%) = 115 (73, M⁺ - 15), 98 (27), 87 (43), 83 (100), 55 (73), 45 (29), 43 (85), 29 38), 27 (55).

Der vorstehende Ester *ent*-**26** (Schema 5) wurde hydriert, wie für *rac*-**20** analog beschrieben. Das erhaltene Produkt enthielt gemäß GC/MS-Analyse den anti-Ester *ent*-**27** mit 96 % *de.*
(2R,3R)-3-Hydroxy-2-methyl-buttersäuremethylester (*ent*-**27**):
¹H-NMR (300 MHz, CDCl₃): δ = 1.15 (t, J = 7 Hz, 3 H), 1.195 (t, J = 6.5 Hz, 3 H), 2.49 (dq, J = 7, 7 Hz, 1 H), 3.55 (br. s, 1 H), 3.70 (s, 3 H), 3.915 ppm (dq (6 Linien), J = 6.5, 7 Hz, 1 H).
¹³C-NMR (75 MHz, CDCl₃): δ = 13.5 (q), 20.4 (q), 47.2 (d), 51.7 (q), 69.2 (d), 176.2 ppm (s).
MS: m/z (%) = 117 (9, M⁺- 15), 101 (19), 88 (100), 87 (15), 85'(21), 59 (12), 57 (52), 56 (25), 55 (16), 45 (24), 43 (12).

### Tetrahydropyranylether (ent-23) von (2R,3R)-4-Brom-3-methylbutan-2-ol (Schema 5)

Die Darstellung erfolgte analog zur vorstehend beschriebenen, vierstufigen Reaktionssequenz aus (2R,3R)-3-Hydroxy-2-methyl-buttersäuremethylester *(ent-***27**).

### 1.3 Synthesen von erfindungsgemäßen Isomeren und Vergleichsisomeren

### Alkoholgemisch rac-A/rac-31-Z/rac-B/rac-30-Z (Schema 6)

a) In einer Stickstoffatmosphäre wurden 87 mg (12,5 mmol) Lithium (feingeschnittenes Granulat) in 5 ml abs. Tetrahydrofuran portionsweise in 2 h bei 20 - 25°C unter Rühren mit einer Lösung von 0,74 g (5 mmol) Keton *rac*-**13** und 1,38 g (5,5 mmol) Bromid *rac*-**23** in 2,5 ml abs. Tetrahydrofuran versetzt und über Nacht bei gleicher Temperatur weiter-gerührt. Der Ansatz wurde zur Entfernung von überschüssigem Lithium über Glaswolle filtriert, dann mit Eiswasser hydrolysiert und in Diethylether aufgenommen. Die wässerige Phase wurde abgetrennt; nach Einengen der organischen Phase am Rotationsverdampfer verblieben 1,6 g Rohgemisch der Alkohole *rac*-**28** und *rac-***29***.*
   MS (beispielhaftes Isomer): m/z (%) = 302 (2, M⁺ - 18), 219 (14), 218 (13), 191 (22), 149 (12), 105 (11), 91 (10), 85 (100), 84 (10), 79 (11), 67 (24).
b) Eine Lösung von 1,6 g des vorstehenden rohen Alkoholgemisches in 25 ml abs. Pyridin wurde bei 0°C unter Rühren mit 7,67 g Phosphoroxychlorid versetzt und über Nacht bei 20°C nachgerührt. Das Reaktionsgut wurde vorsichtig auf Eiswasser gegossen und dann in Diethylether aufgenommen. Die wässerige Phase wurde abgetrennt; die organische Phase wurde mit 10 %iger Schwefelsäure gewaschen, mit Sodalösung neutralgewaschen und am Rotationsverdampfer vom Lösungsmittel befreit. Es verblieben 1,5 g rohes THP-Ether-gemisch der Alkohole *rac*-**A**/*rac***-31-Z**/*rac*-**B**/*rac*-**30-Z**.
   MS (beispielhaftes Isomer): m/z (%) = 302 (Spur, M⁺), 174 (5), 146 (5), 107 (10), 86 (5), 85 (100), 79 (7), 67 (17), 41 (5).
c) Eine Lösung von 1,5 g des vorstehenden rohen THP-Ethergemisches in 15 ml abs. Methanol, 1 g Molekularsieb 3 Å und 50 mg p-Toluolsulfonsäure wurden 2 h bei 20 - 25°C gerührt. Das Reaktionsgut wurde filtriert, dann mit 0,1 g Natriumcarbonat versetzt, und das Methanol am Rotationsverdampfer abdestilliert. Der Rückstand wurde in Wasser und Diethylether aufgenommen. Die wässerige Phase wurde abgetrennt; die organische Phase wurde am Rotationsverdampfer vom Lösungsmittel befreit. Das erhaltene Rohprodukt (1,1 g) wurde an Kieselgel 60 (Fa. Merck; Hexan/Diethylether 7 : 3) chromatografiert; es resultierten 0,62 g Alkoholgemisch *rac*-**A**/*rac*-**31-Z**/*rac*-**B**/*rac*-**30-Z**.
Gaschromatogramm (60 m HP5, 60 - 250°C 4°C/min):
t_{R} = 30.80 (*rac*-**30-Z**), 30.95 (*rac*-**31-Z**), 31.00 (*rac*-**B** = *rac*-**31-E**), 31.05 min (*rac*-**A** = *rac*-**30-E**).

Das E/Z-Verhältnis wurde durch Integration der charakteristischen ¹H-NMR-Signale für das Seitenketten-Vinylproton bei 5.08 (E-Isomere) und 4.85 ppm (Z-Isomere) mit 55 : 45 bestimmt.
NMR-Analyse ergab
(2SR,3RS)-3-Methyl-4-4[(E,1RS,2RS,6RS,7RS)tricyclo[5.2.1.0^{2,6}]dec-4-en-8-yliden]butan-2-ol (*rac*-**A** = *rac*-**30**-**E**) und
(2RS,3SR)-3-Methyl-4-[(Z,1RS,2RS,6RS,7RS)tricyclo[5.2.1.0^{2,6}]dec-4-en-8-yliden]butan-2-ol (*rac*-**31-Z**) als Hauptisomere und *rac*-**B** (= *rac*-**31-E**) und *rac*-**30-Z** als Nebenisomere: ;

Spektrale Daten siehe unter Alkoholgemisch **A/30-Z** und unter Alkoholgemisch **B/31-Z.**

### Alkoholgemisch rac-C/rac-33-Z/rac-D/rac-32-Z (Schema 6)

a) Eine Lösung von 0,1 g Alkoholgemisch *rac*-**A**/*rac*-**31-Z**/*rac*-**B**/*rac*-**30-Z**, 0,22 g Triphenylphosphin und 0,06 g Benzoesäure in 4 ml abs. Tetrahydrofuran wurde unter Rühren bei 20°C mit 0,1 g Azodicarbonsäurediethylester versetzt und 3 d bei 20 - 25°C nachgerührt. Das Reaktionsgut wurde in Wasser und Diethylether aufgenommen. Die wässerige Phase wurde abgetrennt; die organische Phase wurde mit 10 %iger Schwefelsäure und mit Sodalösung gewaschen und am Rotationsverdampfer vom Lösungsmittel befreit. Das erhaltene Rohprodukt (0,45 g) wurde an Kieselgel 60 (Fa. Merck; Hexan/Diethylether 7 : 3) chromatografiert; es resultierten 82 mg reines Benzoatgemisch der Alkohole *rac*-**C**/*rac*-**33-Z**/*rac*-**D**/*rac*-**32-Z**.
   MS (beispielhaftes Isomer): m/z (%) = 322 (Spur, M⁺), 200 (38), 173 (22), 134 (27), 133 (100), 107 (26), 106 (16), 105 (85), 91 (17), 79 (12), 77 (31), 67 (32).
b) Eine Lösung von 82 mg des vorstehenden Benzoatgemisches in 3 ml Methanol und 25 mg 50 %ige Natronlauge wurden 2 h unter Rühren refluxiert. Der Ansatz wurde am Rotations-verdampfer vom Methanol befreit; der Rückstand wurde in Wasser und Dichlormethan aufgenommen. Die wässerige Phase wurde abgetrennt; die organische Phase wurde dreimal mit Wasser gewaschen und am Rotationsverdampfer vom Lösungsmittel befreit. Es verblieben 52 mg Alkoholgemisch *rac*-**C**/*rac*-**33-Z**/*rac*-**D**/*rac*-**32-Z**.
Gaschromatogramm (60 m HP5, 60 - 250°C 4°C/min):
t_{R} = 31.35 (*rac*-**32-Z**), 31.40 (*rac*-**33-Z**), 31.50 (*rac*-**D** = *rac*-**33-E**), 31.60 min (*rac*-**C** = *rac*-**32-E**).

Das E/Z-Verhältnis wurde durch Integration der charakteristischen ¹H-NMR-Signale für das Seitenketten-Vinylproton bei 5.09 (E-lsomere) und 4.86 ppm (Z-Isomere) mit 55 : 45 bestimmt.
NMR-Analyse ergab
(2RS,3RS)-3-Methyl-4-[(E,1RS,2RS,6RS,7RS)tricyclo[5.2.1.0^{2,6}]dec-4-en-8-yliden]butan-2-ol (*rac*-**C** = *rac*-**32-E**) und
(2SR,3SR)-3-Methyl-4-[(Z,1RS,2RS,6RS,7RS)tricyclo[5.2.1.0^{2,6}]dec-4-en-8-yliden]butan-2-ol (*rac*-**33-Z**) als Hauptisomere und *rac*-**D** (= rac-33-**E**) und *rac*-**32-Z** als Nebenisomere:
Spektrale Daten siehe unter Alkoholgemisch **C/32-Z** und unter Alkoholgemisch D/33-Z.

### Alkoholgemisch rac-36-E/rac-37-Z/rac-37-E/rac-36-Z (Schema 7)

Die Darstellung erfolgte analog zur vorstehenden Darstellung des Alkoholgemisches *rac*-**A**/*rac*-**31-Z**/*rac*-**B**/*rac*-**30-Z**, d.h. aus Keton *rac*-**15** und Bromid *rac*-**23** via Kopplungsalkoholgemisch *rac*-**34**/*rac*-**35** und dessen Dehydratisierung und Entschützung.
Gaschromatogramm (60 m DBWax-60N, 50 - 250°C 4°C/min):
t_{R} = 40.50 (*rac*-**36-Z**), 40.55 (*rac*-**37-Z**), 40.85 (*rac*-**37-E**), 40.95 min (*rac*-**36-E**).

Das E/Z-Verhältnis wurde durch Integration der charakteristischen ¹H-NMR-Signale für das Seitenketten-Vinylproton bei 5.08 (E-lsomere) und 4.835 ppm (Z-Isomere) mit 55 : 45 bestimmt.
NMR-Analyse ergab
(2SR,3RS)-3-Methyl-4-[(E,1RS,2SR,6RS,7RS)tricyclo[5.2.1.0^{2,6}]dec-3-en-8-yliden]butan-2-ol (*rac*-**36-E**) und
(2RS,3SR)-3-Methyl-4-[(Z,1RS,2SR,6RS,7RS)tricyclo[5.2.1.0^{2,6}]dec-3-en-8-yliden]butan-2-ol (*rac*-**37-Z**) als Hauptisomere und *rac*-**37-E** und *rac*-**36-Z** als Nebenisomere:
¹H-NMR (300 MHz, CDCl₃):
δ = 3.37 - 3.53 (m, 1 H), 5.47 - 5.53 (m, 1 H), 5.65 - 5.71 ppm (m, 1 H).
*rac*-**36-E** zugeordnet: 8 = 0.925 (d, J = 7 Hz), 1.15 (d, J = 6 Hz), 2.35 (br. s), 5.08 ppm (dt, J = 10, 2.25 Hz).
*rac*-**37-Z** zugeordnet: δ = 0.96 (d, J = 7 Hz), 1.17 (d, J = 6 Hz), 2.68 (br. s), 4.835 ppm (d, J = 10 Hz).
¹³C-NMR (75 MHz, CDCl₃):
*rac*-**36-E** zugeordnet: δ = 16.8 (q), 20.0 (q), 32.45 (t), 35.75 (t), 39.05 (t), 40.9 (d), 41.7 (d), 43.8 (d), 51.8 (d), 55.2 (d), 71.7 (d), 119.7 (d), 132.25 (d), 132.35 (d), 148.25 ppm (s).
*rac*-**37-Z** zugeordnet: δ = 17.4 (q), 19.9 (q), 32.35 (t), 38.35 (t), 39.2 (t), 40.6 (d), 42.45 (d), 43.3 (d), 47.05 (d), 55.1 (d), 71.55 (d), 120.5 (d), 132.15 (d), 132.4 (d), 147.75 ppm (s).
Die Nebenisomere *rac*-**37-E** und *rac*-**36-Z** werden durch Signale geringerer Intensität angezeigt.
MS:
*rac*-**36-E**: m/z (%) = 218 (4, M⁺), 174 (58), 173 (82), 131 (20), 117 (22), 107 (100), 105 (28), 91 (55), 79 (53), 77 (27), 67 (56).
*rac*-**37-Z:** m/z (%) = 218 (2, M⁺), 174 (72), 173 (98), 131 (27), 117 (35), 107 (100), 105 (35), 91 (66), 79 (74), 77 (34), 67 (77).

### Alkoholgemisch rac-38-E/rac-39-Z/rac-39-E/rac-38-Z (Schema 7)

Die Darstellung erfolgte analog zur vorstehenden Darstellung des Alkoholgemisches *rac*-**C**/*rac*-**33-Z**/*rac*-**D**/*rac*-**32-Z** aus Alkoholgemisch *rac*-**36-E**/*rac*-**37-Z**/*rac-***37-E**/*rac*-**36-Z**.
Gaschromatogramm (60 m DBWax-60N, 60 - 250°C 4°C/min):
t_{R} = 40.85 (*rac*-**38-Z**), 41.00 (*rac*-**39-Z**), 41.45 (*rac*-**39-E**), 41.475 min (*rac*-**38-E**).

Das E/Z-Verhältnis wurde durch Integration der charakteristischen ¹H-NMR-Signale für das Seitenketten-Vinylproton bei 5.085 (E-Isomere) und 4.85 ppm (Z-Isomere) mit 55 : 45 bestimmt.
NMR-Analyse ergab
(2RS,3RS)-3-Methyl-4-[(E,1RS,2SR,6RS,7RS)tricyclo[5.2.1.0^{2,6}]dec-3-en-8-yliden]butan-2-ol (*rac*-**38-E**) und
12SR,3SR)-3-Methyl-4-[(Z,1RS,2SR,6RS,7RS)tricyclo[5.2.1.0^{2,6}]dec-3-en-8-yliden]butan-2-ol (*rac*-**39-Z**) als Hauptisomere und *rac*-**39-E** und *rac*-**38-Z** als Nebenisomere:
¹H-NMR (300 MHz, CDCl₃):
δ = 3.54 - 3.64 (m, 1 H), 5.48 - 5.54 (m, 1 H), 5.65 - 5.71 ppm (m, 1 H).
*rac*-**38-E** zugeordnet: δ = 0.965 (d, J = 7 Hz), 1.12 (d, J = 6 Hz), 2.34 (br. s), 5.085 ppm (ddd, J = 10, 5, 2 Hz).
*rac*-**39-Z** zugeordnet: δ = 1.00 (d, J = 7 Hz), 1.115 (d, J = 6.5 Hz), 2.67 (br. s), 4.85 ppm (d, J = 9.5 Hz).
¹³C-NMR (75 MHz, CDCl₃):
*rac*-**38-E** zugeordnet: δ = 16.6 (q), 20.15 (q), 32.3 (t), 35.75 (t), 39.1 (t), 40.8 (d), 40.95 (d), 43.85 (d), 51.8 (d), 55.2 (d), 72.05 (d), 119.3 (d), 132.25 (d), 132.4 (d), 146.85 ppm (s).
*rac*-**39-Z** zugeordnet: δ = 17.3 (q), 19.95 (q), 32.25 (t), 38.4 (t), 39.2 (t), 40.65 (d), 41.25 (d), 43.35 (d), 47.0 (d), 55.1 (d), 72.0 (d), 119.75 (d), 132.2 (d), 132.45 (d), 146.45 ppm (s).

Die Nebenisomere *rac*-**39-E** und *rac*-**38-Z** wurden durch Signale geringerer Intensität angezeigt.
MS:
*rac*-**38-E**: m/z (%) = 218 (2, M⁺), 174 (58), 173 (83), 131 (24), 117 (26), 107 (100), 105 (27), 91 (56), 79 (56), 77 (25), 67 (67).
*rac*-**39-Z**: m/z (%) = 218 (2, M⁺), 174 (54), 173 (86), 131 (24), 117 (25), 107 (100), 105 (28), 91 (62), 79 (57), 77 (27), 67 (69).

### Alkoholgemisch A/30-Z (Schema 8)

Die Darstellung erfolgte analog zur vorstehenden Darstellung des Alkoholgemisches *rac-***A**/*rac-***31-Z**/*rac-***B**/*rac-***30-Z***,* d.h. aus Keton **13** und Bromid **23** via Kopplungsalkohol **28** und dessen Dehydratisierung und Entschützung. Gaschromatogramm (60 m HP5, 60 - 250°C 4°C/min):
t_{R} = 30.80 (19 % **30-Z**), 31.05 min (81 % **A**).
(2S,3R)-3-Methyl-4-[(E,1R,2R,6R,7R)tricyclo[5.2.1.0^{2,6}]dec-4-en-8-yliden]butan-2-ol **(A = 30-E),**
(2S,3R)-3-Methyl-4-[(Z,1R,2R,6R,7R)tricyclo[5.2.1.0^{2,6}]dec-4-en-8-yliden]butan-2-ol **(30-Z):**
¹H-NMR (300 MHz, CDCl₃):
δ = 3.41 - 3.55 (m, 1 H), 5.44 - 5.50 (m, 1 H), 5.64 - 5.70 ppm (m, 1 H).
**A** (= **30-E):** δ = 0.925 (d, J = 7 Hz), 1.145 (d, J = 6 Hz), 2.41 (br. s, 0.8 H), 5.08 ppm (d"d", J = 10, 2.5 Hz, 0.8 H).
**30-Z:** δ = 0.94 (d, J = 7 Hz), 1.17 (d, J = 6 Hz), 4.85 ppm (d, J = 10 Hz, 0.2 H). ¹³C-NMR (75 MHz, CDCl₃):
**A** (= **30-E**): δ = 16.8 (q), 20.0 (q), 32.4 (t), 35.7 (t), 39.7 (t), 41.65 (d), 43.15 (d), 43.2 (d), 49.3 (d), 55.8 (d), 71.7 (d), 119.6 (d), 131.4 (d), 132.25 (d), 148.45 ppm (s).
**30-Z**: δ = 17.3 (q), 19.95 (q), 32.3 (t), 38.15 (t), 39.75 (t), 42.35 (d), 42.95 (d), 43.15 (d), 44.35 (d), 55.2 (d), 71.55 (d), 120.4 (d), 131.4 (d), 132.35 (d), 147.5 ppm (s).
MS:
**A** (= **30-E):** m/z (%) = 218 (8, M⁺), 174 (17), 173 (53), 151 (18), 108 (14), 107 (100), 91 (22), 79 (24), 67 (55).
**30-Z**: m/z (%) = 218 (8, M⁺), 174 (15), 173 (49), 151 (16), 108 (13), 107 (100), 91 (22), 79 (23), 67 (57).

### Alkoholgemisch C/32-Z (Schema 8)

Die Darstellung erfolgte analog zur vorstehenden Darstellung des Alkoholgemisches *rac*-**C**/*rac*-**33-Z**/*rac*-**D**/*rac*-**32-Z** aus Alkoholgemisch **A/30-Z** Gaschromatogramm (60 m HP5, 60 - 250°C 4°C/min):
t_{R} = 31.35 (20 % **32-Z**), 31.55 min (80 % C).
(2R,3R)-3-Methyl-4-[(E,1R,2R,6R,7R)tricyclo[5.2.1.0^{2,6}]dec-4-en-8-yliden]butan-2-ol (**C** = **32-E**),
(2R,3R)-3-Methyl-4-[(Z,11R,2R,6R,7R)tricyclo[5.2.1.0^{2,6}]dec-4-en-8-yliden]butan-2-ol (**32-Z**):
¹H-NMR (300 MHz, CDCl₃):
δ = 3.54 - 3.66 (m, 1 H), 5.44 - 5.50 (m, 1 H), 5.65 - 5.71 ppm (m, 1 H).
**C** (= **32-E):** δ = 0.965 (d, J = 7 Hz), 1.12 (d, J = 6 Hz), 2.39 (br. s, 0.8 H), 5.09 ppm (dt, J = 10,2 Hz, 0.8 H)
**32-Z**: δ = 0.985 (d, J = 7 Hz), 1.16 (d, J = 6 Hz), 4.86 ppm (d, J = 9.5 Hz, 0.2 H) ¹³C-NMR (75 MHz, CDCl₃):
**C** (= **32-E**): δ = 16.65 (q), 20.15 (q), 32.25 (t), 35.7 (t), 39.7 (t), 40.8 (d), 43.15 (d), 43.25 (d), 49.3 (d), 55.85 (d), 72.05 (d), 119.15 (d), 131.5 (d), 132.25 (d), 147.1 ppm (s).
**32-Z**: δ = 17.3 (q), 20.55 (q), 32.3 (t), 38.15 (t), 39.7 (t), 41.55 (d), 42.95 (d), 43.15 (d), 44.3
(d), 55.0 (d), 72.0 (d), 119.9 (d), 131.35 (d), 132.45 (d), 146.2 ppm (s).
MS:
**C** (= **32-E**): m/z (%) = 218 (8, M⁺), 174 (17), 173 (52), 151 (14), 108 (14), 107 (100), 91 (26), 79 (30), 67 (67).
**32-Z**: m/z (%) = 218 (5, M⁺), 174 (15), 173 (47), 151 (12), 108 (12), 107 (100), 91 (25), 79 (30), 67 (72).

### Alkoholgemisch B/31-Z (Schema 9)

Die Darstellung erfolgte analog zur vorstehenden Darstellung des Alkoholgemisches *rac*-**A**/*rac*-**31-Z**/*rac*-**B**/*rac*-**30-Z**, d.h. aus Keton **13** und Bromid *ent*-**23** via Kopplungsalkohol **29** und dessen Dehydratisierung und Entschützung.

Gaschromatogramm (60 m HP5, 60 - 250°C 4°C/min):
t_{R} = 30.95 (70 % **31-Z**), 31.00 min (30 % **B**).
(2R,3S)-3-Methyl-4-[(E,1R,2R,6R,7R)tricyclo[5.2.10^{2,6}]dec-4-en-8-yliden]butan-2-ol (**B**=**31-E**),
(2R,3S)-3-Methyl-4-[(Z,1R,2R,6R,7R)tricyclo[5.2.1.0^{2,6}]dec-4-en-8-yliden]butan-2-ol (**31-Z**):
¹H-NMR (300 MHz, CDCl₃):
δ = 3.38 - 3.53 (m, 1 H), 5.44 - 5.50 (m, 1 H), 5.65 - 5.71 ppm (m, 1 H).
**B** (= **31-E**): δ = 0.925 (d, J = 7 Hz, 0.9 H), 1.155 (d, J = 6 Hz), 2.42 (br. s), 5.075 ppm (dt, J = 10,2 Hz, 0.3 H).
**31-Z**: δ = 0.97 (d, J = 7 Hz, 2.1 H), 1.17 (d, J = 6 Hz), 2.34 (ddq (10 Linien), J = 7, 7, 10 Hz, 0.7 H), 2.73 (br. s), 4.85 ppm (d, J = 10 Hz, 0.7 H) ¹³C-NMR (75 MHz, CDCl₃):
**B** (= **31-E**): δ = 16.7 (q), 19.95 (q), 31.95 (t), 35.45 (t), 39.65 (t), 41.45 (d), 43.1 (d), 43.15 (d), 49.5 (d), 56.4 (d), 71.65 (d), 119.35 (d), 131.35 (d), 132.3 (d), 148.65 ppm (s).
**31-Z:** δ = 17.4 (q), 19.95 (q), 32.35 (t), 38.3 (t), 39.7 (t), 42.25 (d), 42.9 (d), 43.05 (d), 44.55 (d), 55.25 (d), 71.5 (d), 120.25 (d), 131.25 (d), 132.45 (d), 147.85 ppm (s).
MS:
**B** (= **31-E**): m/z (%) = 218 (7, M⁺), 174 (12), 173 (40), 151 (18), 108 (13), 107 (100), 91 (21), 79 (24), 67 (51).
**31-Z**: m/z (%) = 218 (8, M⁺), 174 (19), 173 (58), 151 (12), 108 (14), 107 (100), 91 (25), 79 (28), 67 (61).

### Alkoholgemisch D/33-Z (Schema 9)

Die Darstellung erfolgte analog zur vorstehenden Darstellung des Alkoholgemisches *rac*-**C**/*rac*-**33-Z**/*rac*-**D**/*rac*-**32-Z** aus Alkoholgemisch **B/31-Z** Gaschromatogramm (60 m HP5, 60 - 250°C 4°C/min):
t_{R} = 31.40 (70 % **33-Z**), 31.50 min (30 % **D).**
(2S,3S)-3-Methyl-4-[(E,1R,26R,7R)tricyclo[5.2.1.0^{2,6}]dec-4-en-8-yliden]butan-2-ol (D= **33-E**),
(2S,3S)-3-Methyl-4-[(Z,1R,2R,6R,7R)tricyclo[5.2.1.0.^{2,6}]dec-4-en-8-yliden]butan-2-ol (**33-Z**):
¹H-NMR (300 MHz, CDCl₃):
δ = 3.54 - 3.64 (m, 1 H), 5.44 - 5.50 (m, 1 H), 5.66 - 5.72 ppm (m, 1 H).
**D** (= **33-E):** δ = 0.965 (d, J = 7 Hz, 0.9 H), 1.14 (d, J = 6.5 Hz), 2.40 (br. s), 5.07 ppm (dt, J = 10, 2.25 Hz, 0.3 H.
**33-Z**: δ = 1.005 (d, J = 7 Hz, 2.1 H), 1.12 (d, J = 6.5 Hz), 2.50 (ddq (10 Linien), J = 6.5, 6.5, 10 Hz), 2.73 (br. s), 4.86 ppm (d, J = 10 Hz, 0.7 H).
¹³C-NMR (75 MHz, CDCl₃):
**D** (= **33-E**): δ = 16.65 (q), 20.35 (q), 32.0 (t), 35.55 (t), 39.7 (t), 40.8 (d), 43.15 (d), 43.2 (d), 49.45 (d), 56.05 (d), 72.25 (d), 119.2 (d), 131.4 (d), 132.35 (d), 147.25 ppm (s).
**33-Z**: δ = 17.35 (q), 19.95 (q), 32.25 (t), 38.3 (t), 39.7 (t), 41.2 (d), 42.95 (d), 43.05 (d), 44.55 (d), 55.3 (d), 72.0 (d), 119.5 (d), 131.3 (d), 132.45 (d), 146.75 ppm (s).
MS:
**D (= 33-E)**: m/z (%) = 218 (6, M⁺), 174 (12), 173 (43), 151 (14), 108 (13), 107 (100), 91 (24), 79 (28), 67 (68).
**33-Z**: m/z (%) = 218 (7, M'), 174 (18), 173 (56), 151 (11), 108 (14), 107 (100), 91 (24), 79 (31), 67 (75).

### Alkoholgemisch ent-A/ent-30-Z (Schema 10)

Die Darstellung erfolgte analog zur vorstehenden Darstellung des Alkoholgemisches *rac*-**A**/*rac*-**31-Z**/*rac*-**B**/*rac*-**30-Z**, d.h. aus Keton *ent*-**13** und Bromid *ent*-**23** via Kopplungsalkohol *ent*-**28** und dessen Dehydratisierung und Entschützung. Gaschromatogramm (60 m HP5, 60 - 250°C 4°C/min):
t_{R} = 30.80 (20 % *ent-***30-Z**)*,* 31.05 min (80 % *ent*-**A**).
(2R,3S)-3-Methyl-4-[(E,S.2S.6S,7S)tricyclo[5.2.1.0^{2,6}]dec-4-en-8-yliden]butan-2-ol (*ent*-**A**),
2R,3S)-3-Methyl-4-[(Z,1S,2S,6S,7S)tricyclo[5.2.1.0^{2,6}]dec-4-en-8-yliden]butan-2-ol *ent*-**30-Z** :

Die spektralen Daten entsprechen denen von Alkoholgemisch **A/30-Z.**

### Alkoholgemisch ent-C/ent-32-Z (Schema 10)

Die Darstellung erfolgte analog zur vorstehenden Darstellung des Alkoholgemisches *rac*-**C**/*rac*-**33-Z**/*rac*-**D**/*rac*-**32-Z** aus Alkoholgemisch *ent*-**A**/*ent*-**30-Z** Gaschromatogramm (60 m HP5, 60 - 250°C 4°C/min):
t_{R} = 31.35 (20 % *ent-***32-Z***),* 31.55 min (80 % *ent*-**C***).*
(2S,3S)-3-Methyl-4-[(E,1S,2S,6S,7S)tricyclo[5.2.1.0^{2,6}]dec-4-en-8-yliden]butan-2-ol (*ent*-**C**),
(2S,3S)-3-Methyl-4-[(Z,1S,2S,6S,7S)tricyclo[5.2.1.0^{2,6}]dec-4-en-8-yliden]butan-2-ol (*ent*-**32-Z)**:

Die spektralen Daten entsprechen denen von Alkoholgemisch **C/32-Z.**

### Alkoholgemisch ent-B/ent-31-Z (Schema 11)

Die Darstellung erfolgte analog zur vorstehenden Darstellung des Alkoholgemisches *rac-***A**/*rac-***31-Z**/*rac-***B**/*rac-***30-Z*,*** d.h. aus Keton *ent-***13** und Bromid **23** via Kopplungsalkohol *ent-***29** und dessen Dehydratisierung und Entschützung. Gaschromatogramm (60 m HP5, 60 - 250°C 4°C/min):
t_{R} = 30,95 (71 % *ent-***31-Z**), 31,00 (29 % *ent-***B)***.*
(2S,3R)-3-Methyl-4-[(E,1S,2S,6S,7S)tricyclo[5.2.1.0^{2,6}]dec-4-en-8-yliden]butan-2-ol (ent- **B**),
(2S,3R)-3-Methyl-4-[(Z,1S,2S,6S,7S)tricyclo[5.2.1.0^{2,6}]dec-4-en-8-yliden]butan-2-ol *(ent*-**31-Z**) :

Die spektralen Daten entsprechen denen von Alkoholgemisch **B/31-Z.**

### Alkoholgemisch ent-D/ent-33-Z (Schema 11)

Die Darstellung erfolgte analog zur vorstehenden Darstellung des Alkoholgemisches *rac-C*/*rac-***33-Z**/*rac***-D**/*rac***-32-Z** aus Alkoholgemisch *ent-***B**/*ent-***31*-*Z** Gaschromatogramm (60 m HP5, 60 - 250°C 4°C/min):
t_{R} = 31.40 (71 % *ent*-**33-Z**), 31.50 (29 % *ent*-**D***).*
(2R,3R)-3-Methyl-4-[(E,1S,2S.6S,7S)tricyclo[5.2.1.0^{2,6]}dec-4-en-8-yliden]butan-2-ol (ent-**D**),
(2R,3R)-3-Methyl-4-[(Z,1S,2S,6S,7S)tricyclo[5.2.1.0^{2,6}]dec-4-en-8-yliden]butan-2-ol *(ent-***33-Z)**:

Die spektralen Daten entsprechen denen von Alkoholgemisch **D/33-Z**.

### Beispiel 2: Sensorische Bewertung von erfindungsgemäße und Vergleichsisomeren

### 2.1 (2S,3R)-3-Methyl-4-[(E,1R,2R,6R,7R)tricyclo[5.2.1 .0^{2,6}]dec-4-en-8-yliden]butan-2-ol (A)

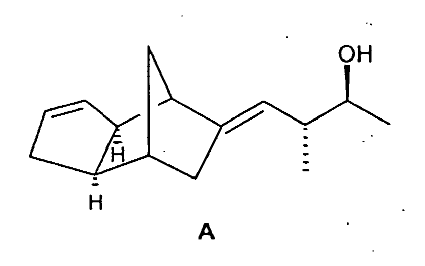

Geruch: sehr stark Sandel, animalisch
Geruchsschwellenwert: 5 µg/l Wasser

### 2.2 (2R,3S)-3-Methyl-4-[(E,1S,2S,6S,7S)tricyclo[5.2.1.0^{2,6}]dec-4-en-8-yliden]butan-2-ol (ent-A) - Vergleich

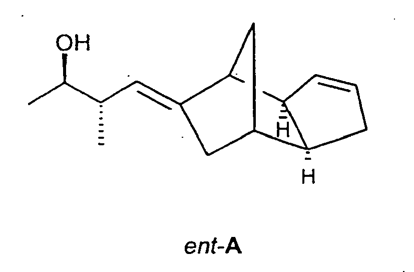

Geruch: schwach holzig
Geruchsschwellenwert: 168 µg/l Wasser

### 2.3 (2R,3S)-3-Methyl-4-[(E,1R,2R,6R,7R)tricyclo[5.2.1.0^{2,6}]dec-4-en-8-yliden]butan-2-ol (B)

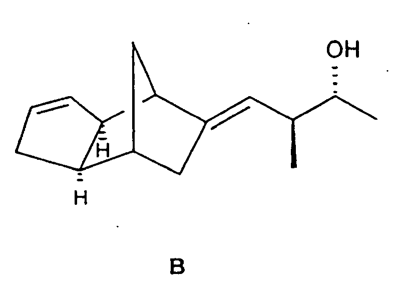

Geruch: Sandel, animalisch
Geruchsschwellenwert: 70 µg/l Wasser

### 2.4 (25,3R)-3-Methyl-4-[(E,1S,2S,6S,7S)tricyclo[5.2.1.0^{2,6}]dec-4-en-8-yliden]butan-2-ol (ent-B) - Vergleich

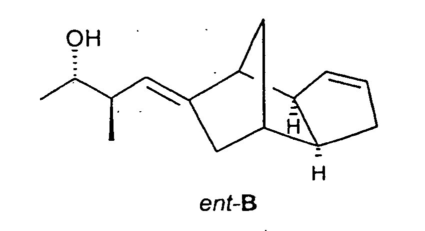

Geruch: holzig
Geruchsschwellenwert: 72 µg/l Wasser

### 2.5 (2R,3R)-3-Methyl-4-[(E,1R,2R,6R,7R)tricyclo[5.2.1.0^{2,6}]dec-4-en-8-yliden]butan-2-ol (C)

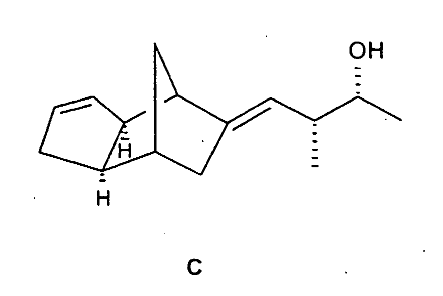

Geruch: Sandel, blumig
Geruchsschwellenwert: 137 µg/l Wasser

### 2.6 (25,3S)-3-Methyl-4-[(E,1S,2S,6S,7S)tricyclo[5.2.1.0^{2,6}]dec-4-en-B-yliden]butan-2-ol (ent-C) - Vergleich

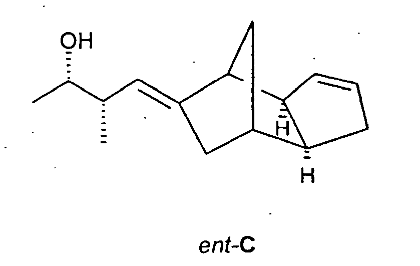

Geruch: sehr schwach blumig
Geruchsschwellenwert: 591 µg/l Wasser

### 2.7 (2S,3S)-3-Methyl-4-[(E,1R,2R,6R,7R)tricyclo[5.2.1.0^{2,6}]dec-4-en-8-yliden]butan-2-ol (D)

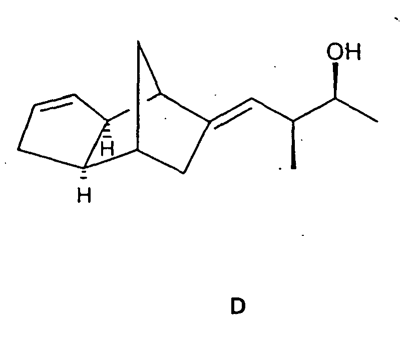

Geruch: stark Sandel, blumig
Geruchsschwellenwert: 46 µg/l Wasser

### 2.8 (2R,3R)-3-Methyl-4-[(E,1S,2S,6S,7S)tricyclo[5.2.1.0^{2,6}]dec-4-en-8-yliden]butan-2-ol (ent-D) - Vergleich

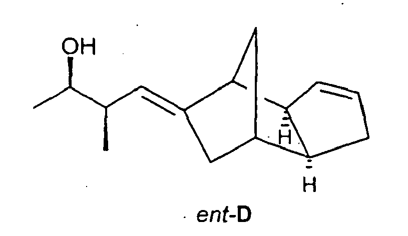

Geruch: schwach blumig
Geruchsschwellenwert: 204 µg/l Wasser

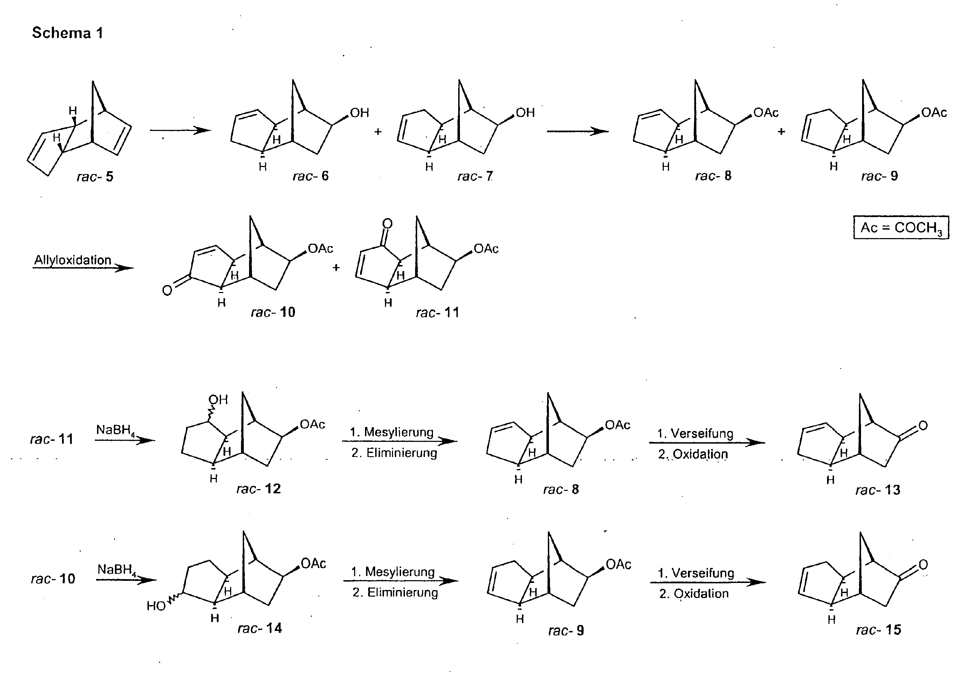

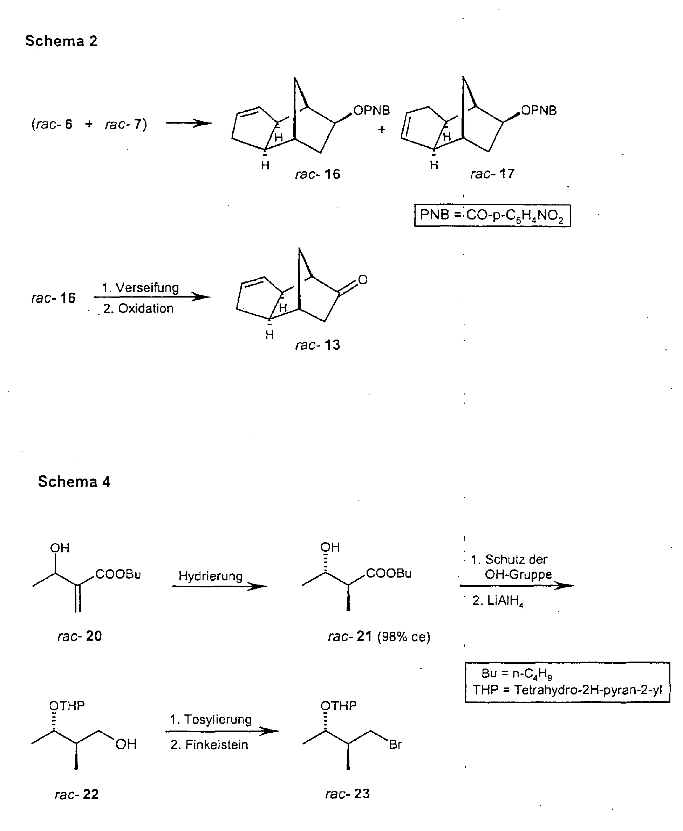

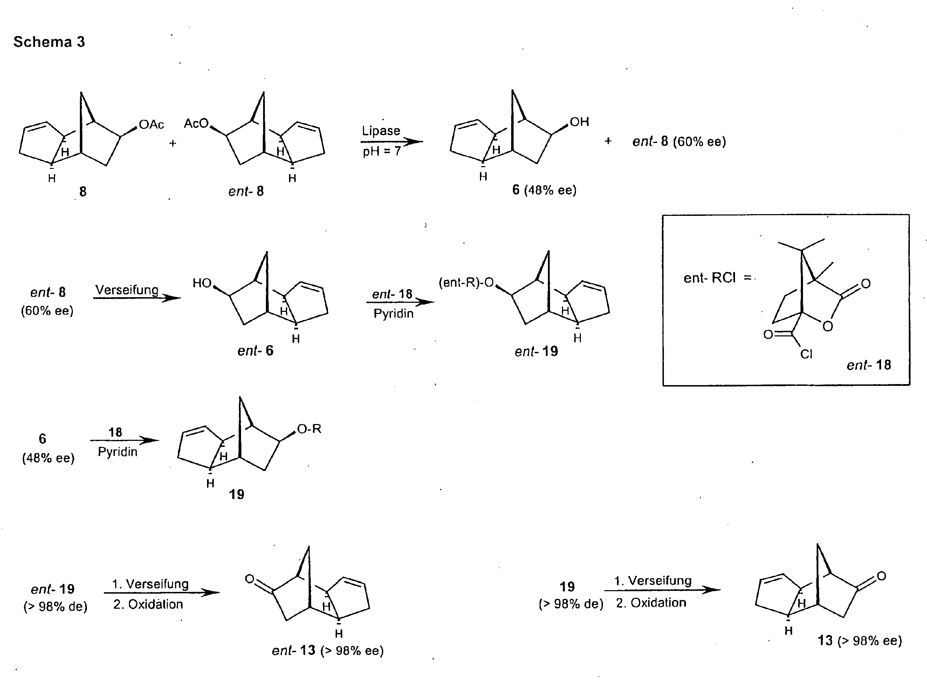

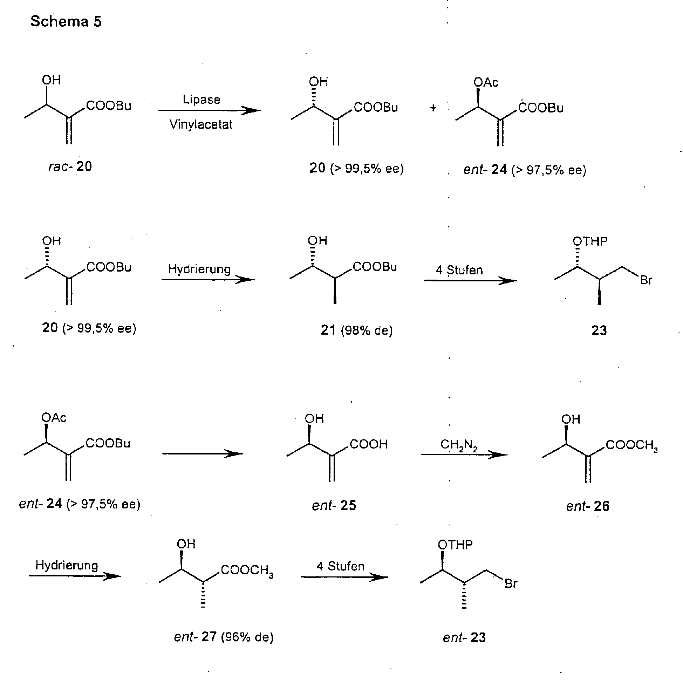

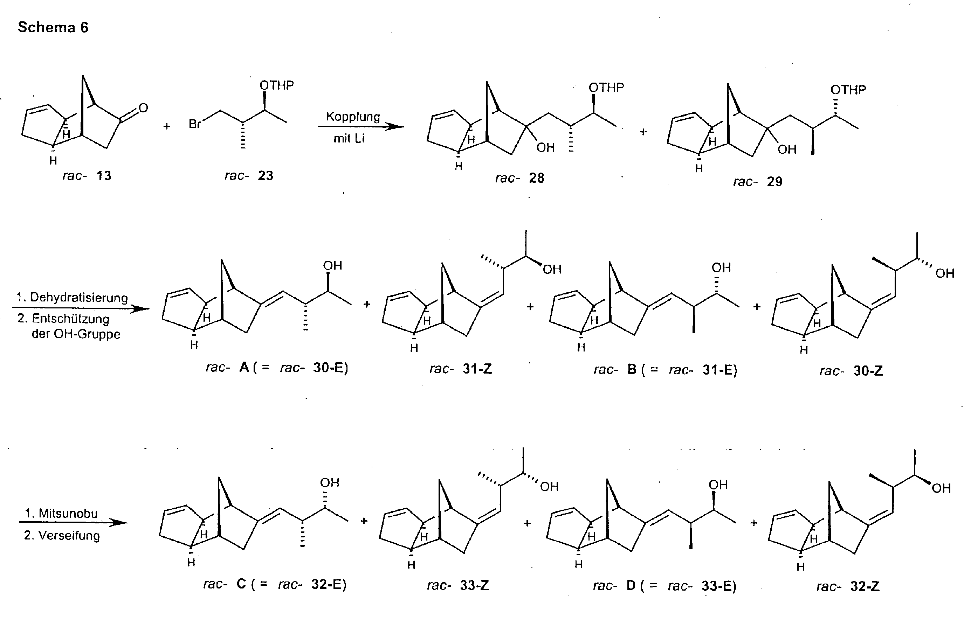

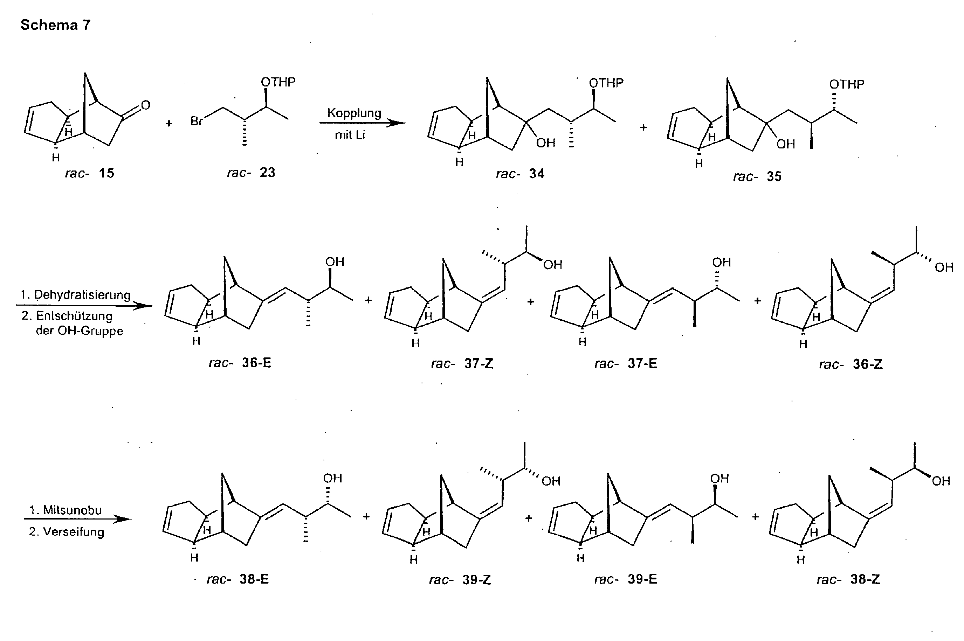

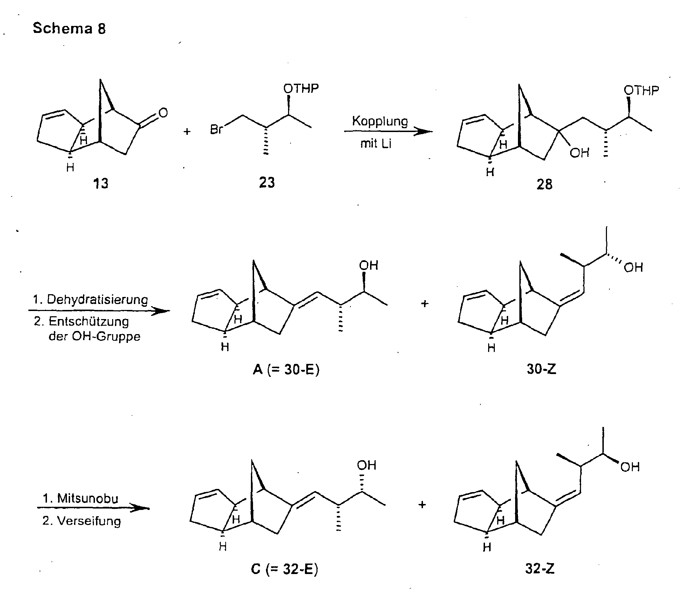

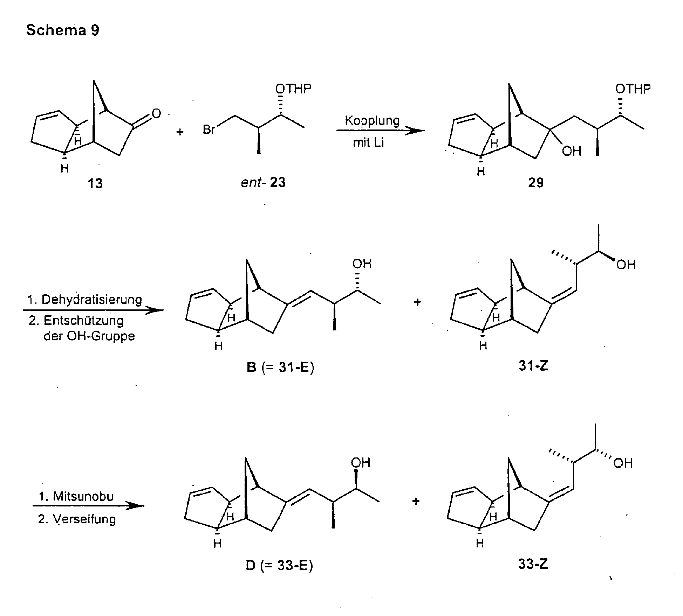

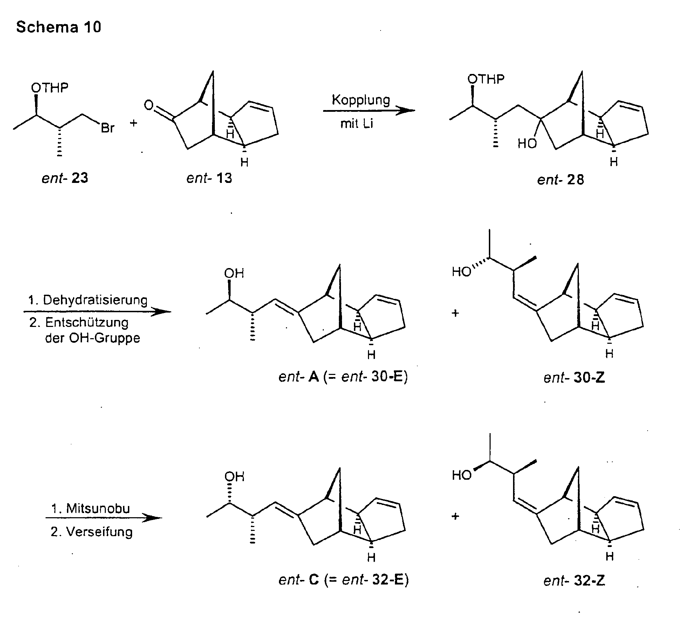

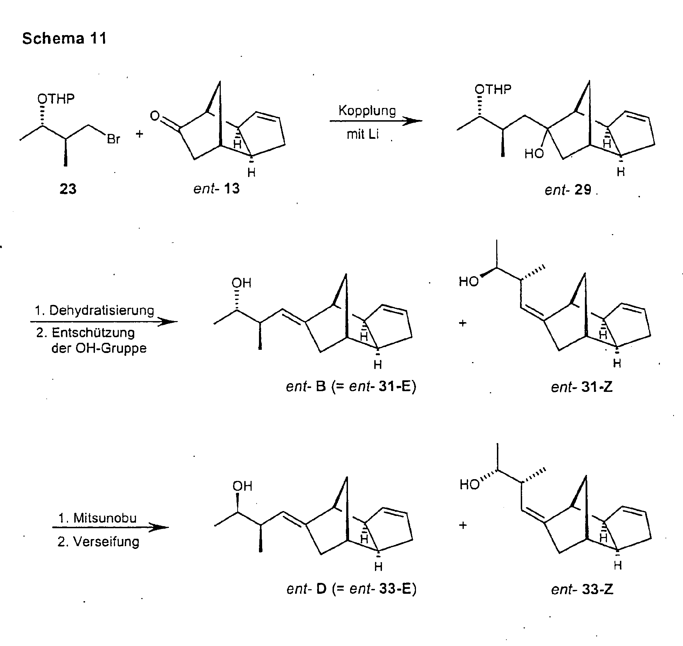

## Patentansprüche

1. Verbindung der Formel
(2R/S, 3R/S)-3-Methyl-4-[(E,1R,2R,6R,7R)tricyclo[5.2.1.0^{2,6}]dec-4-en-8-yliden]butan-2-ol,
wobei jeweils unabhängig voneinander gilt:
- (2R/S, 3R/S) bedeutet (2R,3R), (2R,3S), (2S,3R) oder (2S,3S).

2. Verbindung nach Anspruch 1 mit dem Konfigurationsmerkmal (2S, 3R) oder (2S,3S).

3. Gemisch aus mehreren Konfigurationsisomeren einer Verbindung nach einem der vorangehenden Ansprüche.

4. Gemisch, umfassend
ein Konfigurationsisomer oder mehrere Konfigurationsisomeren einer Verbindung nach einem der Ansprüche 1-2 sowie
eine oder mehrere Verbindungen der Formel
3-Methyl-4-[(E,1R/S,2S/R,6R/S,7R/S)tricyclo[5.2.1.0^{2,6}]dec-3-en-8-yliden]-butan-2-ol
oder der Formel
3-Methyl-4-[(Z,1R/S,2R/S,6R/S,7R/S)tricyclo[5.2.1.0^{2,6}]dec-4-en-8-yliden]-butan-2-ol
oder der Formel
3-Methyl-4-[(Z,1R/S,2S/R,6R/S,7R/S)tricyclo[5.2.1.0^{2,6}]dec-3-en-8-yliden]-butan-2-ol
wobei das Mischungsverhältnis so gewählt ist, dass die sensorischen Eigenschaften des Gemisches überwiegend durch das oder die Konfigurationsisomeren der Verbindung nach einem der Ansprüche 1-2 bestimmt werden.

5. Duftstoff- oder Aromakomposition, umfassend eine sensorisch wirksame Menge einer Verbindung nach einem der Ansprüche 1-2 oder eines Gemisches nach einem der Ansprüche 3-4.

6. Verwendung einer Verbindung nach einem der Ansprüche 1-2 oder eines Gemisches nach einem der Ansprüche 3-4 als Duft- oder Aromastoff.

7. Verfahren zum Modifizieren der sensorischen Eigenschaften einer Duftstoff- oder Aromakomposition, wobei eine oder mehrere Komponenten der Duftstoff- oder Aromakomposition mit einer sensorisch wirksamen Menge einer Verbindung nach einem der Ansprüche 1-2 oder eines Gemisches nach einem der Ansprüche 3-4 vermischt wird.

8. Duftstoffkomposition, umfassend eine Verbindung nach Anspruch 1.

## Claims

1. Compound having the formula
(2R/S, 3R/S)-3-methyl-4-[(E,1R,2R,6R,7R) tricyclo [5.2.1.0^{2,6}]dec-4-en-8-ylidene]butan-2-ol, wherein mutually independently:
- (2R/S, 3R/S) denotes (2R,3R), (2R, 3S), (2S, 3R) or (2S, 3S) .

2. Compound according to claim 1 having the configurational feature (2S, 3R) or (2S, 3S).

3. Mixture of several configurational isomers of a compound according to one of the preceding claims.

4. Mixture comprising
a configurational isomer or several configurational isomers of a compound according to one of claims 1 to 2 and
one or more compounds having the formula
3-methyl-4-[(E,1R/S,2S/R,6R/S,7R/S)tricyclo[5.2.1.0^{2,6}]dec-3-en-8-ylidene]butan-2-ol
or the formula
3-methyl-4-[(Z,1R/S,2S/R,6R/S,7R/S)tricyclo[5.2.1.0^{2,6}]dec-4-en-8-ylidene]butan-2-ol
or the formula
3-methyl-4-[(Z,1R/S,2R/S,6R/S,7R/S)tricyclo[5.2.1.0^{2,6}]dec-3-en-8-ylidene]butan-2-ol
wherein the mixing ratio is chosen such that the sensory characteristics of the mixture are predominantly determined by the configuration isomer(s) of the compound according to one of claims 1 to 2.

5. Perfume or flavouring composition comprising a sensorially effective amount of a compound according to one of claims 1 to 2 or a mixture according to one of claims 3 to 4.

6. Use of a compound according to one of claims 1 to 2 or a mixture according to one of claims 3 to 4 as a perfume or flavouring.

7. Process for modifying the sensory characteristics of a perfume or flavouring composition, wherein one or more components of the perfume or flavouring composition are mixed with a sensorially effective amount of a compound according to one of claims 1 to 2 or a mixture according to one of claims 3 to 4.

8. Perfume composition comprising a compound according to claim 1.

## Revendications

1. Composé de formule
(2R/S,3R/S)-3-méthyl-4-[(E,1R,2R,6R,7R)-tricyclo-[5.2.1.0^{2,6}]-déc-4-èn-8-ylidène]-butan-2-ol,
où, respectivement, indépendamment les uns des autres :
- (2R/S,3R/S) signifie (2R,3R), (2R,3S), (2S,3R) ou (2S,3S).

2. Composé selon la revendication 1 avec la caractéristique de configuration (2S,3R) ou (2S,3S).

3. Mélange de plusieurs isomères de configuration d'un composé selon l'une des revendications précédentes.

4. Mélange comprenant :
un isomère de configuration ou plusieurs isomères de configuration d'un composé selon l'une des revendications 1 à 2 et
un ou plusieurs composés de formule
3-méthyl-4-[(E,1R/S,2S/R,6R/S,7R/S)-tricyclo-[5.2.1.0^{2,6}]-déc-3-èn-8-ylidène]-butan-2-ol,
ou de formule
3-méthyl-4-[(Z,1R/S,2R/S,6R/S,7R/S)-tricyclo-[5.2.1.0^{2,6}]-déc-4-èn-8-ylidène]-butan-2-ol,
ou de formule
3-méthyl-4-[(Z,1R/S,2R/S,6R/S,7R/S)-tricyclo-[5.2.1.0^{2,6}]-déc-3-èn-8-ylidène]-butan-2-ol,
où le rapport de mélange est choisi de sorte que les propriétés sensorielles du mélange soient principalement déterminées par l'isomère ou les isomères de configuration du composé selon l'une des revendications 1 à 2.

5. Composition parfumée ou aromatique, comprenant une quantité sensoriellement efficace d'un composé selon l'une des revendications 1 à 2 ou d'un mélange selon l'une des revendications 3 à 4.

6. Utilisation d'un composé selon l'une des revendications 1 à 2 ou d'un mélange selon l'une des revendications 3 à 4 comme parfum ou arôme.

7. Procédé de modification des propriétés sensorielles d'une composition parfumée ou aromatique, dans lequel un ou plusieurs composants de la composition parfumée ou aromatique sont mélangés avec une quantité sensoriellement efficace d'un composé selon l'une des revendications 1 à 2 ou d'un mélange selon l'une des revendications 3 à 4.

8. Composition parfumée comprenant un composé selon la revendication 1.
